(19) Europäisches Patentamt
European Patent Office
Office européen des brevets

(11) **EP 4 600 649 A1**

(12) **EUROPEAN PATENT APPLICATION**
published in accordance with Art. 153(4) EPC


(43) Date of publication:
**13.08.2025 Bulletin 2025/33**

(21) Application number: **23874972.5**

(22) Date of filing: **06.10.2023**

(51) International Patent Classification (IPC):
***G01N 33/531*** *(2006.01)* ***C08F 220/10*** *(2006.01)*
***C08F 230/02*** *(2006.01)* ***G01N 33/53*** *(2006.01)*
***G01N 33/542*** *(2006.01)* ***G01N 33/543*** *(2006.01)*
***G01N 33/545*** *(2006.01)*

(52) Cooperative Patent Classification (CPC):
**C08F 220/10; C08F 230/02; G01N 33/53; G01N 33/531; G01N 33/542; G01N 33/543; G01N 33/545**

(86) International application number:
**PCT/JP2023/036644**

(87) International publication number:
**WO 2024/075847 (11.04.2024 Gazette 2024/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.10.2022 JP 2022161609**



(71) Applicant: **Sekisui Medical Co., Ltd.**
**Tokyo 103-0027 (JP)**

(72) Inventors:
- **SASAKI Ryuta**
  **Tokyo 103-0027 (JP)**
- **IWASAKI Manami**
  **Tokyo 103-0027 (JP)**
- **ISHIHARA Yuka**
  **Tokyo 103-0027 (JP)**
- **FUJIMURA Kengo**
  **Tokyo 103-0027 (JP)**

(74) Representative: **Ter Meer Steinmeister & Partner Patentanwälte mbB**
**Nymphenburger Straße 4**
**80335 München (DE)**


(54) **NON-SPECIFIC REACTION INHIBITOR, METHOD FOR USING NON-SPECIFIC REACTION INHIBITOR, METHOD FOR INHIBITING NON-SPECIFIC REACTION, BIOCHEMICAL MEASUREMENT REAGENT, SPECIMEN PRETREATMENT SOLUTION, AND BIOCHEMICAL MEASUREMENT REAGENT KIT**


(57) A non-specific reaction inhibitor, which is a copolymer comprising a constitutional unit A derived from 2-methacryloyloxyethyl phosphorylcholine and exhibiting a specific physical property X measured by Fourier transform infrared spectroscopy according to an attenuated total reflection method (ATR method), the non-specific reaction inhibitor being used in a use application of inhibiting a non-specific reaction when measuring an antibody or an antigen contained in a biological sample by mixing the copolymer with the biological sample.


FIG. 1B

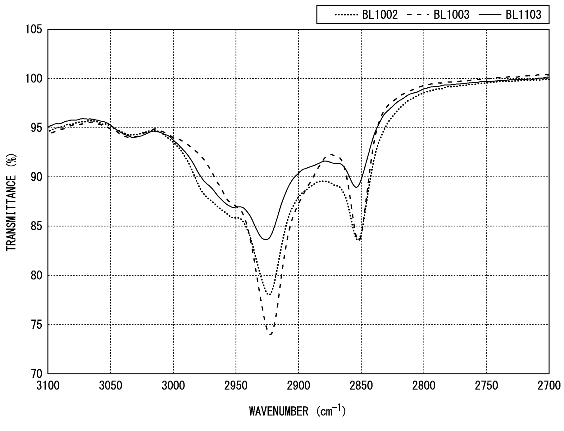


EP 4 600 649 A1

## Description

TECHNICAL FIELD

The present invention relates to a non-specific reaction inhibitor, a method for using a non-specific reaction inhibitor, a method for inhibiting a non-specific reaction, a biochemical measurement reagent, a specimen pretreatment solution, and a biochemical measurement reagent kit. Priority is claimed on Japanese Patent Application No. 2022-161609, filed October 6, 2022, the content of which is incorporated herein by reference.

BACKGROUND ART

In the related art, biochemical measurement methods are known as means for detecting various components contained in a human biological sample. Among the biochemical measurement methods, immunological measurement methods are measurement methods having extremely high specificity because they utilize specific binding between proteins, such as antigen-antibody reactions.

On the other hand, since various substances are present in a sample, binding that is not based on the original specific reaction may occur, or the specific reaction may be hindered. These are referred to as non-specific reactions, and substances causing the non-specific reactions are sometimes referred to as non-specific factors. When a non-specific reaction occurs in a biochemical measurement, a measurement error occurs. For this reason, various non-specific factors have been elucidated so far, and methods for inhibiting non-specific reactions have been investigated.

For example, a heterophile antibody is known as a non-specific factor (Non-Patent Document 1). Heterophile antibody is a general term for human antibodies exhibiting reactivity to animal-derived antibodies, which are main components of immunological measurement methods, and human anti-mouse immunoglobulin antibody (HAMA) is known as a representative example. Furthermore, a rheumatoid factor (RF) is also known as a non-specific factor. Rheumatoid factors are antibodies mainly of the IgM class, which recognize the Fc region of IgG, and are present not only in the blood of patients with rheumatism but also in the blood of healthy individuals. As a method of inhibiting a non-specific reaction caused by a heterophile antibody or a rheumatoid factor, a method of mixing an anti-human IgM antibody, an anti-human IgA antibody, or an anti-human IgG antibody with a sample in advance is known (Patent Document 1). In this way, a component that is added to a reagent in order to inhibit a non-specific reaction is referred to as a non-specific reaction inhibitor.

Meanwhile, a high-molecular-weight polymer having a segment derived from 2-methacryloyloxyethyl phosphorylcholine is a component that is frequently used in immunological measurement reagents. For example, in Patent Document 2, a high-molecular-weight polymer is added to a measurement reagent for the purpose of stabilizing the dispersion of latex particles in a latex turbidimetric immunoassay. Furthermore, the following effects are known: an effect of inhibiting a prozone phenomenon in a latex turbidimetric immunoassay (Patent Document 3); an effect of enabling highly sensitive measurement in an antigen-antibody reaction, an enzymesubstrate reaction, and the like (Patent Document 4); an effect of improving reproducibility in an agglutination immunoassay method (Patent Document 5); and an effect of inhibiting protein adsorption onto a polystyrene plate and the like used in an ELISA method (Patent Document 6). In addition, Patent Document 7 discloses a technology for preventing the occurrence of serum interference by incorporating a copolymer having a segment derived from 2-methacryloyloxyethyl phosphorylcholine and a segment derived from a hydrophilic monomer as a sensitizer into a reagent having a measurement principle based on a latex turbidimetric immunoassay for detecting the presence or absence of an anti-Treponema pallidum antibody in a serum specimen. The types of proteins, lipids, and other various components contained in physiological saline and serum are significantly different. The serum interference described in Patent Document 7 refers to a phenomenon in which, in a case where physiological saline to which an anti-Treponema pallidum antibody standard solution has been added and a syphilis-positive serum to which the same amount of the same standard solution has been added are measured, the measured values are different from each other, and the addition of the above-described components reduces the difference in the measured values.

Citation List

Non-Patent Document

Non Patent Document 1: Biophysical Chemistry 2007, 51, 223-226

Patent Documents

Patent Document 1: Japanese Unexamined Patent Application, First Publication No. H07-012818
Patent Document 2: Japanese Unexamined Patent Application, First Publication No. 2001-228149
Patent Document 3: Japanese Unexamined Patent Application, First Publication No. 2001-318099
Patent Document 4: Japanese Unexamined Patent Application, First Publication No. 2002-022740
Patent Document 5: PCT International Publication No. WO 2002/018953
Patent Document 6: Japanese Unexamined Patent Application, First Publication No. 2015-148558
Patent Document 7: Japanese Patent No. 4629564

## SUMMARY OF INVENTION

### Technical Problem

The method of inhibiting a non-specific reaction using an anti-human IgM antibody or the like as described in Patent Document 1 is a method that is currently being put to practical use with various reagents. However, it has been revealed by the tests of the present inventors that there is a non-specific reaction that cannot be inhibited even by these antibodies. Those non-specific reactions could not be inhibited even when the components described in Patent Document 7 were used. An object of the present invention is to also inhibit such a non-specific reaction.

An object of the present invention is to provide a non-specific reaction inhibitor capable of inhibiting a non-specific reaction and improving measurement accuracy, a method for using a non-specific reaction inhibitor, a method for inhibiting a non-specific reaction, a biochemical measurement reagent, a specimen pretreatment solution, and a biochemical measurement reagent kit.

### Solution to Problem

[1] A non-specific reaction inhibitor, which is a copolymer comprising a constitutional unit A derived from 2-methacryloyloxyethyl phosphorylcholine and exhibiting the following physical property X, in which the non-specific reaction inhibitor is used in a use application of inhibiting a non-specific reaction when measuring a measurement target substance contained in a biological sample by mixing the copolymer with the biological sample.

[2] A method for using a non-specific reaction inhibitor, the method including: using a copolymer comprising a constitutional unit A derived from 2-methacryloyloxyethyl phosphorylcholine and exhibiting the following physical property X, as a non-specific reaction inhibitor, in which a non-specific reaction is inhibited when measuring a measurement target substance contained in a biological sample by mixing the copolymer with the biological sample.

[3] A method for inhibiting a non-specific reaction, the method including: inhibiting a non-specific reaction when measuring a measurement target substance contained in a biological sample by mixing a copolymer comprising a constitutional unit A derived from 2-methacryloyloxyethyl phosphorylcholine and exhibiting the following physical property X, with the biological sample.

[4] A biochemical measurement reagent, which is a reagent used for biochemical measurement, in which the reagent contains a copolymer comprising a constitutional unit A derived from 2-methacryloyloxyethyl phosphorylcholine and exhibiting the following physical property X.

[5] The biochemical measurement reagent according to [4], in which the biochemical measurement reagent is a reagent used for biochemical measurement, the reagent contains a copolymer comprising a constitutional unit A derived from 2-methacryloyloxyethyl phosphorylcholine and exhibiting the following physical property X, and the copolymer is contained as a non-specific reaction inhibitor that inhibits a non-specific reaction in a biological sample.

[6] The biochemical measurement reagent according to either [4] or [5], in which the biochemical measurement is a homogeneous method.

[7] The biochemical measurement reagent according to any one of [4] to [6], in which the biochemical measurement is a measurement based on an antigen-antibody reaction.

[8] The biochemical measurement reagent according to any one of [4] to [7], in which the biochemical measurement is a latex turbidimetric immunoassay.

[9] The biochemical measurement reagent according to any one of [4] to [8], in which a biological sample as a measurement target of the biochemical measurement is a blood sample.

[10] The biochemical measurement reagent according to any one of [4] to [9], in which the biochemical measurement is detection of an antigen-antibody reaction between any one or more selected from a soluble interleukin 2 receptor and a pulmonary surfactant protein D, which are contained in a biological sample as a measurement target, and an antibody added to the biological sample.

[11] A specimen pretreatment solution, which is a reagent used for biochemical measurement, in which the reagent

contains a copolymer comprising a constitutional unit A derived from 2-methacryloyloxyethyl phosphorylcholine and exhibiting the following physical property X, and a liquid medium in which the copolymer is dissolved or dispersed.

[12] A biochemical measurement reagent kit, including the biochemical measurement reagent according to any one of [4] to [10] or the specimen pretreatment solution according to [11].

[13] A method for using a biochemical measurement reagent, the method including: mixing the biochemical measurement reagent according to any one of [4] to [10] or the specimen pretreatment solution according to [11] with a biological sample, and performing biochemical measurement.

[14] A method for inhibiting a non-specific reaction by a biochemical measurement reagent, in which when biochemical measurement is performed by mixing the biochemical measurement reagent according to any one of [4] to [10] or the specimen pretreatment solution according to [11] with a biological sample, a non-specific reaction in the biological sample is inhibited by the copolymer.

<Physical property X>

In a spectrum of Fourier transform infrared spectroscopy according to an attenuated total reflection method (ATR method), there is a peak having a maximum absorption at 2870 to 2833 $cm^{-1}$.

Advantageous Effects of Invention

According to the present invention, a non-specific reaction inhibitor that can inhibit a non-specific reaction and improve measurement accuracy, a method for using a non-specific reaction inhibitor, a method for inhibiting a non-specific reaction, a biochemical measurement reagent, a specimen pretreatment solution, and a biochemical measurement reagent kit are provided.

In addition, when the non-specific reaction inhibitor of the present invention is used, it is possible to inhibit only a non-specific reaction without reducing the measurement sensitivity.

The phrase "inhibiting a non-specific reaction" described in the present specification means a state in which a non-specific reaction derived from a sample, which is caused for some reason, is reduced, and a measured value is brought closer to an original measured value (true value). The true value is a measured value obtained by a reference measurement method in a case where a reference measurement method is available for the measurement target component. For a measurement target component for which a reference measurement method is not available, it is considered that a measurement by a heterogeneous method (measurement method in which a washing step and a B/F separation step are carried out during measurement) is generally less likely to cause a non-specific reaction than a measurement by a homogeneous method (measurement method in which a washing step and a B/F separation step are not carried out during measurement). Even in the case of the heterogeneous method, the true value may not be obtained due to the influence of the non-specific reaction. In that case, the sample is diluted with a component that does not contain a measurement target component, such as physiological saline, the measurement is performed again, and it is determined whether the measured value is a value according to the dilution ratio. Thereby, the degree of influence of the non-specific reaction can be figured out, and the true value can be obtained.

In the case of inhibiting non-specific adsorption, means for coating all the components involved in the reaction with some kind of component may be considered. For example, a case where a non-specific adsorption inhibitor is used corresponds to this case. A non-specific adsorption inhibitor can be fixed to and coat the surface of the inner wall of a reagent container, the inner wall of a reaction container (for example, the inner wall of a microplate used in the ELISA method, the inner wall of a reaction cell of an automatic analyzing apparatus, or the like), a membrane or a pad in immunochromatography, and the like. Furthermore, a non-specific adsorption inhibitor can block a solid phase (a microplate, latex particles, magnetic particles, fluorescent particles, a membrane in immunochromatography, or the like) to which an antigen or an antibody is bound. When such a non-specific adsorption inhibitor is added to a biochemical measurement reagent, a biochemical reaction that is originally desired to occur may not easily occur, and the measurement sensitivity may be decreased. In the present specification, it is intended to inhibit a non-specific reaction that is difficult to inhibit with a non-specific adsorbent without causing a decrease in such measurement sensitivity.

BRIEF DESCRIPTION OF DRAWINGS

[FIG. 1A] A view showing infrared absorption spectra of various products comprising a constitutional unit A derived from 2-methacryloyloxyethyl phosphorylcholine, which are obtained by a Fourier transform infrared spectroscopy (FT-IR) according to an attenuated total reflection method (ATR method), and are spectra of Lipidures used in Examples 1 to 3.

[FIG. 1B] An enlarged view of a portion of the spectra of Lipidures, which are copolymers used in Examples 1 to 3.
[FIG. 1C] An enlarged view of a portion of the spectra of Lipidures, which are copolymers used in Examples 1 to 3.
[FIG. 1D] An enlarged view of a portion of the spectra of Lipidures, which are copolymers used in Examples 1 to 3.
[FIG. 1E] Spectra of Lipidures, which are copolymers used in Comparative Examples 3 to 4 and Comparative Examples 6 to 10.
[FIG. 1F] An enlarged view of a portion of spectra of Lipidures, which are copolymers used in Comparative Examples 3 to 4 and Comparative Examples 6 to 10.
[FIG. 1G] Spectra of Lipidures, which are copolymers used in Comparative Examples 11 to 16.
[FIG. 1H] An enlarged view of a portion of spectra of Lipidures, which are copolymers used in Comparative Examples 11 to 16.
[FIG. 2] Scatter diagrams showing results of a comparison between an LTIA method and a CLEIA method for the measurement results of soluble interleukin 2 receptor (sIL-2R) in human serum.
[FIG. 3] Scatter diagrams showing results of a comparison between the LTIA method and the CLEIA method for the measurement results of pulmonary surfactant protein D (SP-D) in human serum.

DESCRIPTION OF EMBODIMENTS

<<Non-specific reaction inhibitor>>

A first aspect of the present invention is a copolymer comprising a constitutional unit A derived from 2-methacryloyloxyethyl phosphorylcholine and exhibiting the following physical property X. Here, the constitutional unit may be rephrased as a segment or a repeating unit.

<Physical property X>

In an infrared absorption spectrum obtained by a Fourier transform infrared spectroscopy (FT-IR) according to an attenuated total reflection method (ATR method), there is a peak having a maximum absorption at 2870 to 2833 $cm^{-1}$.

[Infrared absorption spectrum]

An infrared absorption spectrum is a summary of the vibrational frequencies (hereinafter, also simply referred to as "wavenumbers") characteristic of the functional groups generated from each functional group in a molecule when the molecule is irradiated with infrared light, in one diagram. In the present invention, the infrared absorption spectrum is a value measured by a Fourier transform infrared spectroscopy (FT-IR) using an attenuated total reflection method (ATR method). An infrared absorption spectrum is shown as a graph of a curve connecting successive measured values, with the axis of ordinate representing transmittance (%) and the axis of abscissa representing wavenumber (unit: $cm^{-1}$). When a peak having a maximum absorption is present in a specific wavenumber range, the graph changes from decreasing to increasing at that peak.
The numerical values of the infrared absorption spectrum described in the present specification are based on values measured under the following apparatus and measurement conditions. When a measuring apparatus having performance equivalent to or greater than that listed below is used, and equivalent measurement conditions are applied, the same measurement results should be obtained.

<Measuring apparatus>

Nicolet™ iS5 FT-IR manufactured by Thermo Fisher Scientific, Inc.

<Measurement conditions>

Crystal: Diamond
Angle of incidence: 45°
Correction: None

In the first aspect of the present invention, the infrared absorption spectrum of the above-described copolymer exhibits at least two peaks having the maximum absorption in the range of 3000 to 2800 $cm^{-1}$. Among the peaks in this range, the wavenumber range of the peak on the high wavenumber side (hereinafter, may be referred to as P1) and the

wavenumber range of the peak on the low wavenumber side (hereinafter, may be referred to as P2) are as follows.

In one embodiment of the above-described aspect, P1 appears at 3000 to 2880 $cm^{-1}$. The absorption maximum of P1 is in the range of 2946 to 2906 $cm^{-1}$, and preferably in the range of 2936 to 2916 $cm^{-1}$. P2 appears at 2879 to 2800 $cm^{-1}$. The absorption maximum of P2 is in the range of 2870 to 2833 $cm^{-1}$, preferably in the range of 2865 to 2833 $cm^{-1}$, and more preferably in the range of 2863 to 2843 $cm^{-1}$. The number of peaks having a maximum absorption in the range of P1 and the range of P2 is not particularly limited when taking into consideration that the number of peaks may also be affected by the resolution of the infrared absorption spectrometer; however, the number of peaks is preferably 1 to 5, more preferably 1 to 3, even more preferably 1 or 2, and most preferably 1. The transmittance (%) at the absorption maximum may be P1 > P2, may be P1 = P2 or P1 ≈ P2, or may be P1 < P2. It is preferable that P1 < P2.

In general, the region of 3000 to 2800 $cm^{-1}$ is a region in which peaks of CH stretching, $CH_2$ symmetric stretching, $CH_3$ symmetric stretching, $CH_2$ antisymmetric stretching, and $CH_3$ antisymmetric stretching appear, and it is presumed that P1 and P2 belong to at least one or more of these. In particular, $CH_2$ symmetric stretching has a peak near 2853 $cm^{-1}$, and $CH_2$ antisymmetric stretching has a peak near 2926 $cm^{-1}$ (Spectroscopic Identification of Organic Compounds, 8th Edition, Tokyo Kagaku Dojin Publishing Co., Inc., 2016, p. 84). In general, a peak in an infrared absorption spectrum shifts under the influence of a peripheral molecular structure; however, in the region of 3000 to 2800 $cm^{-1}$, the fluctuation of a peak is generally considered to be within a range of ±10 $cm^{-1}$.

In the first aspect of the present invention, the infrared absorption spectrum of the above-described copolymer further exhibits a peak having a maximum absorption in the range of 1000 to 920 $cm^{-1}$ (hereinafter, may be referred to as P3).

In one embodiment of the above-described aspect, the maximum absorption of P3 is in the range of 975 to 940 $cm^{-1}$. The ratio (P1/P3) of the intensity at the maximum absorption of P1 with respect to the intensity at the maximum absorption of P3 (100%) is 16% or more, preferably 20% or more, more preferably 26.6% or more, even more preferably 27% or more, and most preferably 28% or more. The ratio (P2/P3) of the intensity at the maximum absorption of P2 with respect to the intensity at the maximum absorption of P3 (100%) is 8% or more, preferably 10% or more, more preferably 12% or more, even more preferably 15% or more, and still more preferably 18% or more. There is a possibility that P3 may be a peak attributable to a -P-O-C- structure included in the constitutional unit A (Japanese Unexamined Patent Application, First Publication No. 2002-143294).

In the first aspect of the present invention, the infrared absorption spectrum of the copolymer further exhibits a peak in the range of 1300 to 1200 $cm^{-1}$ (hereinafter, may be referred to as P4).

In one embodiment of the aspect, the maximum absorption of P4 is in the range of 1252 to 1215 $cm^{-1}$. The ratio (P1/P4) of the intensity at the maximum absorption of P1 with respect to the intensity at the maximum absorption of P4 (100%) is 18% or more, preferably 25% or more, more preferably 28.4% or more, even more preferably 29% or more, and most preferably 30% or more. The ratio (P2/P4) of the intensity at the maximum absorption of P2 with respect to the intensity at the maximum absorption of P4 (100%) is 9% or more, preferably 12% or more, more preferably 15% or more, and even more preferably 20% or more. There is a possibility that P4 may be a peak attributable to P=O included in the constitutional unit A (Japanese Unexamined Patent Application, First Publication No. H3-39309).

In the first aspect of the present invention, the infrared absorption spectrum of the copolymer further exhibits a peak in the range of 1100 to 1000 $cm^{-1}$ (hereinafter, may be referred to as P5). In one embodiment of the aspect, the maximum absorption of P5 is in the range of 1090 to 1040 $cm^{-1}$. There is a possibility that P5 may be a peak attributable to PO-O- included in the constitutional unit A (Japanese Unexamined Patent Application, First Publication No. H10-324634).

The intensity of the infrared absorption spectrum is represented by the absorbance (%) obtained by subtracting the transmittance (%) at each wavenumber obtained by the measurement from 100 (%), as in Formula (1).

$$\text{Intensity of infrared absorption spectrum} = 100\ (\%) - \text{transmittance}\ (\%)\ \text{at the wavenumber} \quad (1)$$

The ratio R of the intensity K2 of the maximum absorption at the second wavenumber with respect to the intensity K1 of the maximum absorption at the first wavenumber is calculated from Formula (2).

$$\text{Ratio R} = \text{Intensity K2/intensity K1} \times 100\ (\%)\ ...\ (2)$$

[Kinematic viscosity]

In the first aspect of the present invention, it is preferable that the kinematic viscosity (unit: $m^2/s$) of an aqueous solution in which the copolymer is dissolved in water at a concentration of 5% by mass (hereinafter, also referred to as wt%) is in the following range at 25°C. That is, the kinematic viscosity is preferably in the range of 0.2 to 4.0 $m^2/s$, more preferably in the range of 0.4 to 3.8, and even more preferably 0.6 to 3.6. In addition, in the above-described preferred range, the lower limit value thereof may be 0.8 or more, 1.0 or more, 1.2 or more, or 1.4 or more. Furthermore, the upper limit value thereof

may be 3.0 or less, 2.7 or less, or 2.4 or less.

These lower limit values and upper limit values can be arbitrarily combined, and the kinematic viscosity can be, for example, 1.4 to 2.4.

The kinematic viscosity refers to a value obtained by dividing the viscosity by the density of the liquid under the same conditions (temperature and pressure). A method for measuring the viscosity is defined in Japanese Industrial Standards JIS Z 8803. The kinematic viscosity can be measured using a capillary viscometer. A person ordinarily skilled in the art can purchase any measuring instrument and measure the kinematic viscosity. The measuring instrument is sold by, for example, Sibata Scientific Technology, Ltd. and A&D Co., Ltd.

[Surface tension]

It is preferable that the surface tension (unit: $\times 10^{-3}$ N/m) of an aqueous solution in which the above-described copolymer is dissolved in water at a concentration of 0.1 wt% be in the following range at 25°C. That is, the surface tension is preferably in the range of $45 \times 10^{-3}$ to $75 \times 10^{-3}$ N/m. In this preferred range, the lower limit value thereof may be 47 or more, or 49 or more. Furthermore, the upper limit value thereof may be 73 or less, 71 or less, or 69 or less. These lower limit values and upper limit values can be arbitrarily combined, and the surface tension can be, for example, 49 to 69.

Surface tension is expressed as surface energy per unit area. The surface tension can be measured by a Wilhelmy plate method. The measuring instrument is sold by, for example, Kyowa Interface Science Co., Ltd. and Sanyo Trading Co., Ltd.

[Constitutional unit of above-described copolymer]

The constitutional unit A of the above-described copolymer is also present in the copolymer described in Patent Document 7.

It is preferable that the above-described copolymer have one or more kinds of a constitutional unit B, which does not have a functional group that is ionized in water. The constitutional unit B has a structure that gives rise to at least a peak of P2, in the above-described infrared absorption spectrum. When the above-described copolymer has the constitutional unit B, the copolymer described in Patent Document 7 and the copolymer of the present aspect are different from each other from the viewpoint of comprising the constitutional unit B, in addition to the above-described infrared absorption spectrum. By comprising the constitutional unit B, the copolymer of the present aspect exhibits the above-described infrared absorption spectrum (peak P1, peak P2, peak intensity ratios P1/P3, P2/P3, P1/P4, and P2/P4), exhibits a kinematic viscosity and a surface tension in specific ranges, and can further inhibit a non-specific reaction.

The molar fraction of the constitutional unit A with respect to all the constitutional units constituting the copolymer of the present aspect is preferably 2% to 98%, more preferably 10% to 95%, even more preferably 30% to 95%, and most preferably 50% to 90%. In addition, the molar fraction can be set to 60% to 90%, 65% to 90%, 70% to 90%, 70% to 85%, or 75% to 85%.

The total molar fraction of the constitutional unit A and the constitutional unit B with respect to all the constitutional units constituting the copolymer of the present aspect is preferably 60% to 100%, more preferably 80% to 100%, even more preferably 85% to 100%, and most preferably 90% to 100%. In a case where the total molar fraction is 100%, the copolymer is composed only of the constitutional unit A and the constitutional unit B.

When each constitutional unit is in the range of the above-described molar fraction, a non-specific reaction can be even further inhibited.

The ratio of the constitutional unit A to the constitutional unit B in the copolymer of the present aspect can be, for example, 1:9 to 9:1, can be 1:8 to 8:1, can be 1:7 to 7:1, or can be 1:6 to 6:1, in terms of molar ratio. Furthermore, the ratio can be set to 1:5 to 5:1, can be set to 1:4 to 4:1, or can be set to 7:3 to 3:7. Furthermore, the ratio can be set to 1:1 to 6:1, can be set to 2:1 to 6:1, can be set to 3:1 to 6:1, or can be set to 4:1 to 6:1. When the ratio is in the above-described range, a non-specific reaction can be even further inhibited.

The molar fraction of the constitutional unit B with respect to the remainder obtained by excluding the constitutional unit A from all the constitutional units constituting the copolymer of the present aspect can be set to 10% to 100%, can be set to 20% to 100%, can be set to 40% to 100%, or can be set to 50% to 100%. Furthermore, the molar fraction is preferably 60% to 100%, and more preferably 70% to 100%. The molar fraction can be set to 80% to 100%, can be set to 90% to 100%, or can be set to 95% to 100%. Furthermore, the molar fraction is preferably 55% to 95%, 60% to 95%, 65% to 95%, 70% to 95%, 55% to 90%, 60% to 90%, 65% to 90%, 70% to 90%, 55% to 85%, 60% to 85%, 65% to 85%, or 70% to 85%. The above-described remainder may include a constitutional unit C other than the constitutional unit B.

When the constitutional unit B is in the range of the above-described molar fraction, a non-specific reaction can be further inhibited. The ratio of the constitutional unit A to the constitutional unit B in the copolymer of the present aspect, and the molar fraction of the constitutional unit B with respect to the remainder obtained by excluding the constitutional unit A

from all the constitutional units can be measured using, for example, thermolysis gas chromatography mass spectrometry (GC-MS) or nuclear magnetic resonance spectroscopy (1H-NMR, 13C-NMR).

The monomer forming the constitutional unit A has a functional group having a structure represented by Formula (3). In the formula, $R^A$, $R^B$, and $R^C$ are each independently a hydrogen atom, an alkyl group having 1 to 6 carbon atoms, or a hydroxyalkyl group having 1 to 6 carbon atoms; and n is an integer of 1 to 4. Among these, a structure in which $R^A$, $R^B$, and $R^C$ are methyl groups and n = 2, is preferred.

[chemical formula 1]

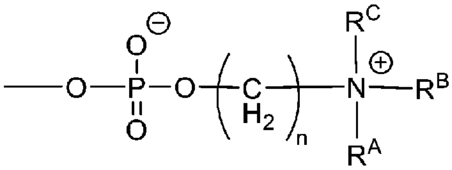

・・・(3)

Specific examples of the monomer having the structure represented by Formula (3) include 2-(meth)acryloyloxyethyl phosphorylcholine represented by Formula (4) (2-methacryloyloxyethyl phosphorylcholine is preferred). In the formula, X represents a hydrogen atom or a methyl group, and a methyl group is preferred. Since Formula (4) has a (meth) acryloyl group, the monomer can be copolymerized with a polymerizable monomer that forms the constitutional unit B. In the present specification, the notation of (meth)acryl is an abbreviation for acryl or methacryl, and the same applies to the notation of other similar terms.

A copolymer of the constitutional unit A and the constitutional unit B, or a copolymer of the constitutional unit A, the constitutional unit B, and the constitutional unit C has a structure exhibiting the above-described infrared absorption spectra P3 and P4.

[chemical formula 2]

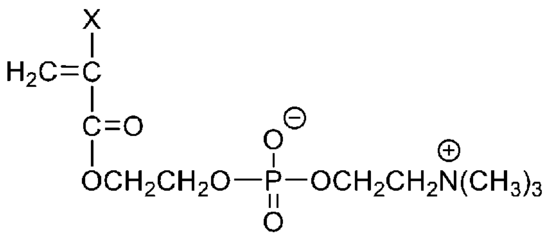

・・・(4)

The constitutional unit B has a maximum absorption at 2870 to 2833 $cm^{-1}$ in the infrared absorption spectrum and does not have a functional group that ionizes in water. Here, the "functional group that ionizes in water" refers to a functional group that can have a positive charge or a negative charge by donating or receiving a proton in water. For example, an acid group such as a carboxy group or a sulfonic acid group is ionized by releasing a proton in water. Furthermore, an amino group is ionized by taking in a proton in water. A hydroxy group of an alcohol does not ionize; however, a phenolic hydroxy group bonded to an aromatic ring ionizes in water.

Examples of a functional group that may be contained in the constitutional unit B and does not ionize in water include an alcoholic hydroxy group and a linear or branched hydrocarbon group which may have a cyclic moiety. The hydrocarbon group may be an aliphatic hydrocarbon group or an aromatic hydrocarbon group, and is preferably an aliphatic hydrocarbon group. Furthermore, a carbonyl group and/or an ether bond may be included. The number of carbon atoms in the hydrocarbon group may be, for example, 1 to 24. In the present specification, a functional group that does not ionize in water may be referred to as a hydrophobic functional group.

Examples of the polymerizable monomer that forms the constitutional unit B include a compound represented by Formula (5). The constitutional unit B may be composed of one kind or two or more kinds of compounds represented by Formula (5).

[chemical formula 3]

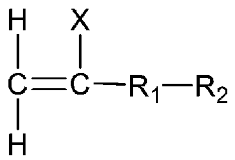

・・・(5)

In the formula, X represents a hydrogen atom or a methyl group, and a methyl group is preferred.

In the formula, $R_1$ represents a group selected from the group consisting of - $C_6H_4$-, -$C_6H_{10}$-, -(C=O)O-, -O-, -(C=O)NH-, -O-(C=O)-, and -O-(C=O)-O-.

In the formula, $R_2$ represents a hydrophobic functional group selected from a hydrogen atom, $-(CH_2)_g-R_3$, and $-((CH_2)_p-O)_n-R_3$. Here, g represents an integer of 7 to 24; h represents an integer of 1 to 24; p represents an integer of 3 to 5; and $R_3$ represents a functional group selected from a hydrogen atom, a methyl group, $-C_6H_5$, and $-O-C_6H_5$. Among these, a structure represented by Formula (6), in which $R_1$ is -(C=O)O-, is preferred. In Formula (6), X is preferably a methyl group.

[chemical formula 4]

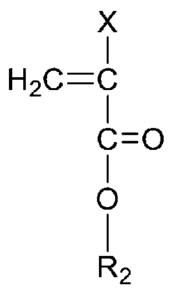

・・・(6)

$R_2$ is preferably a structure having no hydrogen-bonding functional group. The phrase "having no hydrogen-bonding functional group" means that the structure does not have a functional group capable of forming a hydrogen bond with a polar molecule such as water or an alcohol, and/or a functional group capable of forming a hydrogen bond between side chains $R_2$ in the above-described copolymer. Examples of the hydrogen-bonding functional group include a hydroxyl group (-OH), an amino group ($-NH_2$), a carboxyl group (-C(=O)OH), and a phosphate group ($-P(=O)(OH)_2$).

Examples of $R_2$ having no hydrogen-bonding side chain include a linear or branched aliphatic hydrocarbon group, a cyclic alkyl group, and an aromatic group. Among these, since $R_2$ has a maximum absorption in the range of 2870 to 2833 $cm^{-1}$, which is a range showing $CH_2$ symmetric stretching and $CH_2$ antisymmetric stretching in the infrared absorption spectrum, $R_2$ is preferably $-(CH_2)_g-R_3$, and g is preferably 7 or more, 8 or more, 10 or more, or 12 or more. In order to facilitate the dissolution of the copolymer of the present aspect in a buffer solution constituting a biochemical measurement reagent, g can be set to 24 or less, 22 or less, 20 or less, or 18 or less. g can be set to 8 to 24, 10 to 24, or 12 to 24. Furthermore, g can be set to 8 to 22, 10 to 22, or 12 to 22. Furthermore, g can be set to 8 to 20, 10 to 20, or 12 to 20. Furthermore, g can be set to 8 to 18, 10 to 18, or 12 to 18. Among these, g is preferably 12 to 22, 12 to 20, or 12 to 18, and most preferably 12 to 18. $R_3$ is preferably a hydrogen atom.

Specific examples of the polymerizable monomer (hydrophobic monomer) forming the constitutional unit B include a linear or branched alkyl (meth)acrylate having an aliphatic hydrocarbon group, a cyclic alkyl (meth)acrylate, and an aromatic (meth)acrylate. More specific examples thereof include linear or branched alkyl (meth)acrylates having an aliphatic hydrocarbon group, such as octyl (meth)acrylate, decyl (meth)acrylate, lauryl (meth)acrylate, tetradecyl (meth) acrylate, cetyl (meth)acrylate, stearyl (meth)acrylate, oleyl (meth)acrylate, arachidyl (meth)acrylate, and behenyl (meth) acrylate; cyclic alkyl (meth)acrylates such as cyclohexyl (meth)acrylate; aromatic (meth)acrylates such as benzyl (meth) acrylate and phenoxyethyl (meth)acrylate; and hydrophobic polyalkylene glycol (meth)acrylates such as polypropylene glycol (meth)acrylate. The notation of (meth)acryl is an abbreviation for acryl or methacryl. Among these hydrophobic monomers, a linear or branched alkyl (meth)acrylate is preferred, and octyl (meth)acrylate, decyl (meth)acrylate, lauryl (meth)acrylate, tetradecyl (meth)acrylate, cetyl (meth)acrylate, stearyl (meth)acrylate, oleyl (meth)acrylate, arachidyl (meth)acrylate, and behenyl (meth)acrylate are preferred. In addition, lauryl (meth)acrylate, tetradecyl (meth)acrylate, cetyl (meth)acrylate, stearyl (meth)acrylate, and oleyl (meth)acrylate are most preferred.

When the constitutional unit B is a monomer exhibiting a maximum absorption at 2870 to 2833 $cm^{-1}$ in the infrared absorption spectrum, two or more kinds thereof can be used as a mixture.

The constitutional unit C can be used as a constitutional component of the copolymer of the present aspect as desired. The constitutional unit C is a constitutional unit derived from any polymerizable monomer that can be polymerized with the constitutional unit A and the constitutional unit B. The constitutional unit C is neither the constitutional unit A nor the constitutional unit B. The constitutional unit C can have a structure having a functional group that ionizes in water. Furthermore, the constitutional unit C can have a structure having no functional group that ionizes in water. Specific examples of the polymerizable monomer (hydrophobic monomer) forming the constitutional unit C include methyl (meth) acrylate, ethyl (meth)acrylate, and butyl (meth)acrylate.

The constitutional unit C can be copolymerized to the extent that a polymer composed of the constitutional unit A and the constitutional unit B exhibits a practically useful non-specific reaction inhibiting ability. The constitutional unit C can be copolymerized, for example, for the purpose of improving the production stability, the handleability, the storage stability, and the like of a polymer composed of the constitutional unit A and the constitutional unit B.

The weight-average molecular weight of the copolymer of the present aspect is not particularly limited, and the weight-average molecular weight can be set to, for example, 5,000 to 5,000,000, can be set to 10,000 to 4,000,000, can be set to 20,000 to 3,000,000, or can be set to 20,000 to 2,000,000. In addition, the weight-average molecular weight is preferably 20,000 to 1,000,000, 20,000 to 500,000, 20,000 to 200,000, or 20,000 to 100,000. The weight-average molecular weight is most preferably 20,000 to 50,000.

When the molecular weight is within the above-described range, a non-specific reaction can be further inhibited.

When the weight-average molecular weight is 5,000 or more, the affinity with non-specific reaction substances is increased, and the effect of the above-described copolymer as a non-specific reaction inhibitor is likely to be sufficiently obtained. Furthermore, when the weight-average molecular weight is 5,000,000 or less, the viscosity of the reagent according to the present invention containing the above-described copolymer is inhibited from becoming excessively high, and the intended measurement is likely to be smoothly performed.

Regarding the method for measuring the weight-average molecular weight of the copolymer of the present aspect, reference can be made to WO2022/124288.

The copolymer of the present aspect can be synthesized according to the method described in Patent Document 2 (Japanese Unexamined Patent Application, First Publication No. 2001-228149). Furthermore, the copolymer of the present aspect can be purchased as a commercially available product. For example, among various series of Lipidure (registered trademark) sold by NOF Corporation, BL1002, BL1003, and BL1103 of the BL series may be mentioned to be suitable. These can be appropriately combined to the extent that does not affect the stirring, dispersion, and the like of the reagent, in consideration of other components included in the biochemical measurement reagent, for example, the type, pH, and concentration of the buffer solution constituting the reagent, and the type and concentration of the polymer substance other than the present copolymer.

Among various series of Lipidure (registered trademark) sold by NOF Corporation, the following series do not exhibit a non-specific reaction inhibitory effect and do not have the properties inherent to the copolymer of the present aspect.

The BL400 series does not exhibit the above-described physical property X in terms of the infrared absorption spectrum. Furthermore, according to the NOF Corporation website, the side chain property of the constitutional unit B of the BL400 series is anionic, and it is also not preferable from the viewpoint of containing a constitutional unit that ionizes in water, in addition to the constitutional unit A.

The BL500 series does not exhibit the physical property X in terms of the infrared absorption spectrum. Furthermore, according to the above website, the side chain property of the constitutional unit B is cationic, and it is also not preferable from the viewpoint of containing a constitutional unit that ionizes in water, in addition to the constitutional unit A.

The BL800 series does not exhibit the physical property X in terms of the infrared absorption spectrum. Furthermore, according to the above website, the side chain property of the constitutional unit B is considered to be "hydrophilic and hydrophobic", and it is also not preferable from the viewpoint of containing a constitutional unit that ionizes in water, in addition to the constitutional unit A. In addition, it is also not preferable from the viewpoint that the kinematic viscosity is more than 4.0 $m^2/s$ and the surface tension is more than $70 \times 10^{-3}$ N/m.

Furthermore, BL205, BL206, BL1201, BL1202, and BL1301 do not exhibit the physical property X in terms of the infrared absorption spectrum.

Various series of Lipidure (registered trademark) can be used in combination with the copolymer of the present aspect for the purpose of providing effects different from the non-specific reaction inhibitory effect, such as a sensitizing effect, a blocking effect, and a non-specific adsorption inhibitory effect.

<Use application of non-specific reaction inhibitor>

The copolymer of the present aspect is used in a use application of inhibiting a non-specific reaction caused by a sample, when mixing the copolymer with a biological sample (specimen) (hereinafter, may be simply referred to as a

sample) and measuring a measurement target substance contained in the sample. By "inhibiting a non-specific reaction", a measurement error can be reduced when measuring a measurement target substance contained in the above-described sample, and a measured value can be made closer to the true measured value. Examples of the above-described non-specific reaction include a reaction that reduces or inhibits an interaction between components that are originally supposed to interact with each other, a reaction that causes an interaction between components that are originally supposed not to interact with each other, and a reaction that promotes an interaction between components that are originally supposed to interact with each other only to a weak extent that is not supposed to be detected.

The biological sample may be human-derived or animal-derived. The sample may be blood, or may be a culture supernatant, urine, feces, cerebrospinal fluid, saliva, sweat, ascites, an extract of cells or tissues, or the like. The blood may be whole blood, may be plasma, may be serum, or may be a sample obtained by subjecting blood to any treatment such as dilution or purification.

Examples of the measurement target substance include a measurement target substance of an immunological measurement method that will be described later.

<<Method of using non-specific reaction inhibitor>>

A second aspect of the present invention is a method of using a non-specific reaction inhibitor, in which the non-specific reaction inhibitor according to the first aspect is mixed with a sample, thereby inhibiting a non-specific reaction when measuring a measurement target substance contained in the above-described sample. Since the description of the first aspect can also be applied to the second aspect, any description overlapping with the first aspect will not be repeated.

In the method of use of the present aspect, the method of mixing the non-specific reaction inhibitor with the sample is not particularly limited. For example, a method of preparing a reagent S in which the non-specific reaction inhibitor is contained in any buffer solution at a desired concentration, and mixing this with a sample, may be mentioned. The reagent may be a reagent included in a biochemical measurement reagent kit, for example, a first reagent described in Examples of the present specification, or may be a sample diluent or a pretreatment solution.

In a case where the reagent S is a reagent included in a biochemical measurement reagent kit, generally a 10-fold to 100-fold volume of the reagent S is added to the sample. The content concentration of the non-specific reaction inhibitor with respect to the total volume of the reagent S in this case is, for example, preferably 0.001% to 5.00% (w/v), more preferably 0.002% to 1.00% (w/v), even more preferably 0.003% to 0.60% (w/v), particularly preferably 0.005% to 0.50% (w/v), and most preferably 0.04% to 0.30% (w/v).

In a case where the reagent S is a sample diluent or a pretreatment solution, generally a 0.1-fold to 10-fold volume of the reagent S is added to the sample. The content concentration of the non-specific reaction inhibitor with respect to the total volume of the reagent S in this case is, for example, preferably 0.001 % to 5.00% (w/v), more preferably 0.01% to 4.50% (w/v), and even more preferably 0.10% to 3.00% (w/v), 0.20% to 2.50% (w/v), or 0.5% to 2.00% (w/v). When the content concentration is within these suitable ranges, the dispersibility of the non-specific reaction inhibitor in the reagent is increased, and the non-specific reaction inhibitor is likely to be uniformly mixed in the sample.

Regarding a mixed liquid obtained by mixing the non-specific reaction inhibitor with a sample, it is preferable to leave the mixed liquid at, for example, room temperature or 37°C for 5 minutes or more so that the interaction for exerting the non-specific reaction inhibitory effect of the present invention, such as contact and reaction of the non-specific reaction inhibitor with the components contained in the sample, is more likely to occur. When such a mixing time is provided, a non-specific reaction can be sufficiently inhibited when measuring a measurement target substance contained in the above-described sample.

The content concentration of the non-specific reaction inhibitor with respect to the total volume of the above-described mixed liquid is, for example, preferably 0.001% to 5.00% (w/v), more preferably 0.002% to 1.00% (w/v), even more preferably 0.003% to 0.60% (w/v), particularly preferably 0.02% to 0.40% (w/v), and most preferably 0.04% to 0.30% (w/v). When the content concentration is in these suitable ranges, the dispersibility of the non-specific reaction inhibitor in the above-described mixed liquid is increased, and the above-described interaction may occur sufficiently.

<<Method for inhibiting non-specific reaction>>

A third aspect of the present invention is a method for inhibiting a non-specific reaction, in which a non-specific reaction is inhibited when a measurement target substance contained in a sample is measured, by mixing the non-specific reaction inhibitor according to the first aspect with the above-described sample. Since the description of the first and second aspects can also be applied to the third aspect, any overlapping description will not be repeated.

By sufficiently dispersing the non-specific reaction inhibitor in a mixed liquid obtained by mixing the non-specific reaction inhibitor with a sample, a non-specific reaction can be sufficiently inhibited when measuring a measurement target substance contained in the above-described sample.

As an example of the present aspect, an analysis method of performing at least one of a biochemical reaction and

a measurement in the presence of the non-specific reaction inhibitor of the first aspect may be mentioned.

As another example of the present aspect, a biochemical measurement method of biochemically measuring a measurement target substance in a sample, in which a reaction product obtained by a reaction between the measurement target substance and a specific affinity substance is measured in a liquid phase containing the above-described sample in the presence of the non-specific reaction inhibitor of the first aspect, may be mentioned.

Here, the content of the non-specific reaction inhibitor of the first aspect with respect to the total volume of the liquid phase in which the generated reaction product is measured is, for example, preferably 0.0005% to 5.00% (w/v), more preferably 0.001% to 1.00% (w/v), even more preferably 0.003% to 0.60% (w/v), particularly preferably 0.004% to 0.40% (w/v), and most preferably 0.03% to 0.30% (w/v). When the content is in these suitable ranges, the non-specific reaction in the liquid phase to be measured can be sufficiently inhibited. These suitable concentration ranges are also similarly suitable in other examples of the present aspect and in other aspects.

As another example of the present aspect, a biochemical measurement method of measuring a measurement target substance in a sample by a turbidimetric immunoassay such as LTIA, the method including a step of bringing insoluble carrier particles carrying a specific affinity substance for the measurement target substance into contact with the sample in a liquid phase in the presence of the non-specific reaction inhibitor of the first aspect, may be mentioned.

As another example of the present aspect, a biochemical measurement method of measuring a measurement target substance in a sample by a turbidimetric immunoassay such as LTIA, the method including the following steps 1 to 3, may be mentioned.

(Step 1) A step of bringing the sample into contact with the non-specific reaction inhibitor of the first aspect in a liquid phase

(Step 2) A step of adding insoluble carrier particles carrying a specific affinity substance for the measurement target substance, into the liquid phase after the step 1

(Step 3) A step of measuring an agglutination reaction between the measurement target substance and the insoluble carrier particles after the step 2

In the biochemical measurement, a specific affinity substance for a measurement target substance is used. When the measurement target substance is an antibody, an antigen corresponding to the antibody can be used as the specific affinity substance in the above-described step 2. When the measurement target substance is an antigen, an antibody against the antigen can be used as the specific affinity substance. Furthermore, in a case where the measurement target substance is labeled with avidin or biotin, biotin or avidin can be used as the specific affinity substance. Furthermore, when the measurement target substance is a sugar or a sugar chain, a lectin corresponding to the sugar or the sugar chain can be used as the specific affinity substance. In addition to that, an aptamer, an oligonucleotide, and a peptide can also be used as the specific affinity substance. Among these, a measurement that utilizes an antigen-antibody reaction is called an immunological measurement. The "non-specific reaction" described in the present specification is a reaction that interferes with a normal reaction between the measurement target substance and the above-described specific affinity substance. As a component derived from a sample that causes a non-specific reaction interacts with the measurement target substance, interacts with the specific affinity substance, or interacts with both, a measured value of the measurement target substance deviates from the true value.

An example of a more specific measurement method of the present aspect may be a measurement method for measuring sIL-2R in a blood sample by optically measuring or visually observing the degree of agglutination caused by an antigen-antibody reaction between sIL-2R in the blood sample and an insoluble carrier carrying an anti-sIL-2R antibody, in which a sIL-2R measurement reagent including the insoluble carrier carrying an anti-sIL-2R antibody and the non-specific reaction inhibitor of the first aspect is used. Here, sIL-2R is a soluble interleukin 2 receptor.

Furthermore, an example of a more specific measurement method of the present aspect is a measurement method for measuring SP-D in a sample by optically measuring or visually observing a degree of agglutination caused by an antigen-antibody reaction between SP-D in the sample and an insoluble carrier carrying an anti-SP-D antibody, in which an SP-D measurement reagent including the insoluble carrier carrying an anti-SP-D antibody and the non-specific reaction inhibitor of the first aspect is used. Here, SP-D is pulmonary surfactant protein D.

<<Biochemical measurement reagent>>

A fourth aspect of the present invention is a biochemical measurement reagent used for a biochemical measurement for detecting binding that has occurred in a sample between a biochemical measurement target substance and a substance having a specific affinity for the measurement target substance, in which the reagent includes the non-specific reaction inhibitor of the first aspect, and the non-specific reaction inhibitor is included as an active ingredient inhibiting a non-specific reaction in the above-described sample. Since the description of the first to third aspects can also be applied to the fourth aspect, any overlapping description will not be repeated.

A representative example of the above-described binding that has occurred in the above-described sample is an antigen-antibody reaction. In the antigen-antibody reaction, the measurement target substance may be an antigen or may be an antibody. Examples of the binding other than the antigen-antibody reaction include binding between avidin and biotin, binding between protein A and an immunoglobulin Fc region, binding between protein G and an immunoglobulin Fc region, and binding between protein L and an immunoglobulin κ chain.

Hereinafter, as an example of the present aspect, an immunological measurement reagent used for immunological measurement for detecting an antigen-antibody reaction will be described in detail.

The type of immunological measurement is not particularly limited and is applicable to various known methods, and examples thereof include a turbidimetric immunoassay (TIA), a latex turbidimetric immunoassay (LTIA), immunochromatography (lateral flow type, flow-through type), an electrochemiluminescence immunoassay (ECLIA), a chemiluminescence immunoassay (CLIA method), and an enzyme-linked immunosorbent assay (ELISA).

In general, immunological measurements are roughly classified into homogeneous methods and heterogeneous methods. A homogeneous method is one in which a washing treatment (so-called binding (B)/non-binding (F) separation treatment) of separating a reaction product generated by a measurement target substance involved in a mixed solution of the above-described sample and a reagent solution, and another substance (an impurity or the like) contained in the sample, is not performed. According to the reagent of the present aspect, a non-specific reaction in a sample can be inhibited, and the detection accuracy of an antigen-antibody reaction can be increased. For this reason, it is preferable that the reagent of the present aspect be used in a homogeneous method. Furthermore, since the inhibition of the non-specific reaction exhibited by the reagent of the present aspect is also notable in the presence of latex particles similarly to the case of the absence of latex particles, it is more preferable that the reagent of the present aspect be used in LTIA.

An example of the present aspect includes an immunological measurement reagent containing the non-specific reaction inhibitor of the first aspect and a reagent for LTIA.

<LTIA>

Hereinafter, LTIA will be described in detail as an example of the immunological measurement method of the present aspect.

The latex turbidimetric immunoassay (LTIA) is a method of measuring a measurement target substance using latex particles on which a specific affinity substance for the measurement target substance, such as an antigen or an antibody, is immobilized, and the method is widely used in the field of clinical examination.

Methods of measuring an antigen as the measurement target substance by LTIA are roughly classified into the following two types.

(a) A method of causing latex particles on which an antibody against the measurement target substance is immobilized to react with an antigen, which is the measurement target substance, forming a sandwich-type immune complex, and measuring the measurement target substance (antigen) from the degree of agglutination of the latex particles accompanying the formation of the immune complex.

(b) A method of causing latex particles having an antigen immobilized thereon and an antigen (measurement target substance) in a sample to compete with a free antibody added to a separate reagent, inhibiting the formation of an immune complex of the latex particles and the antibody, and measuring the measurement target substance (antigen) from the degree of inhibition of agglutination of the latex particles accompanying the inhibition of formation of the immune complex. In this method, a combination of a free antibody added to the reagent and latex particles on which an antibody against the measurement target substance is immobilized can also be used. Both an antibody and an antigen can be immobilized on the latex particles.

In the LTIA, agglutination that should not occur may occur (positive measurement error), or agglutination that should occur may not occur (negative measurement error) in the latex particles on which the specific affinity substance for the measurement target substance is immobilized, due to any component contained in the sample. These are called non-specific reactions and impair measurement accuracy and reliability.

By inhibiting a non-specific reaction, the measurement error can be reduced, and the positive or negative measurement error of the above-described measured value can be made to approach the original measured value (true value).

The cause of the occurrence of a non-specific reaction is not necessarily clear; however, there is a case where immunoglobulins such as HAMA and a rheumatoid factor, lipoproteins such as very-low-density lipoprotein (VLDL), and lipids such as chylomicron, simple lipids, and triglycerides are considered to be involved. Particularly, a state in which the value of VLDL or chylomicron is high is referred to as "chyle".

In the LTIA, a test substance can be measured by optically or electrochemically observing the degree of agglutination that has occurred. Examples of a method of optically observing the degree of agglutination include methods

of measuring the intensity of scattered light, the absorbance, or the intensity of transmitted light using an optical apparatus (endpoint method, rate method, or the like). The concentration (quantitative value) of the measurement target substance contained in the sample is calculated by comparing a measured value such as an absorbance obtained by measuring the sample with a measured value such as an absorbance obtained by measuring a standard substance (a sample with a known concentration of the measurement target substance). The measurement of the absorbance of transmitted light, scattered light, or the like may be a single-wavelength measurement or a two-wavelength measurement (difference or ratio between two wavelengths). The measurement wavelength is generally selected from 500 to 900 nm.

The measurement of LTIA may be performed by a manual method, or may be performed using a measuring apparatus. The measuring apparatus may be a generalpurpose analyzing apparatus, or may be a dedicated automatic measuring apparatus. The LTIA is generally carried out in a plurality of operation steps such as a two-step method (two-reagent method).

The measurement target substance in the immunological measurement of the present aspect is not particularly limited as long as it is a substance measured by a known immunological measurement method, and examples thereof include proteins (an antigen, a hapten, and an antibody), saccharides, lipids, nucleic acids, and chemical substances (a hormone and a drug). Among them, an antigen or an antibody is preferred, and an antigen composed of a protein is more preferred. Specific examples thereof include soluble interleukin-2 receptor (sIL-2R), CRP, fibrin and fibrinogen degradation products, D-dimer, soluble fibrin (SF), lipoprotein (a) (Lp (a)), matrix metalloproteinase-3 (MMP-3), prostate specific antigen (PSA), IgG, IgA, IgM, IgE, IgD, anti-streptolysin O antibody, rheumatoid factor, transferrin, haptoglobin, α1-antitrypsin, α1-acid glycoprotein, α2-macroglobulin, hemopexin, antithrombin-III, α-fetoprotein, carcinoembryonic antigen (CEA), ferritin, hepatitis B surface antigen (HBs-Ag), anti-hepatitis B surface antibody (anti-HBs), hepatitis B e antigen (HBe-Ag), anti-hepatitis B e antibody (anti-HBe), anti-hepatitis B core antibody (anti-HBc), SARS-CoV-2, human brain natriuretic peptide (BNP), pulmonary surfactant protein D (SP-D), and procalcitonin.

Examples of the specific affinity substance for a measurement target substance carried by insoluble carrier particles such as latex include proteins, peptides, amino acids, lipids, saccharides, nucleic acids, and haptens, all of which are used in known immunological measurement methods. In general, an antibody or an antigen is often used. The above-described antibody may be a polyclonal antibody or a monoclonal antibody.

The above-described antibody may be an entire antibody molecule, or may be a functional fragment having an antigen-antibody reaction activity.

The above-described antibody may be obtained by immunizing an animal such as a mouse, or may be synthesized by protein engineering. Examples of these antibodies include an antibody treated with a proteolytic enzyme, $F(ab')_2$, Fab', a singlechain antibody (scFv), a chimeric antibody, a humanized antibody, and a bispecific antibody (BsAb). The above-described antibodies are obtained by known methods.

The latex particles used in the LTIA are not particularly limited, and latex particles generally used as an immunological measurement reagent can be applied. The latex particles are formed by, for example, polymerizing one or more of the following monomers. Specific examples of the monomer include polymerizable unsaturated aromatics, including polymerizable monomers having a phenyl group, such as styrene, α-methylstyrene, o-methylstyrene, p-methylstyrene, p-chlorostyrene, 4-vinylbenzoic acid, divinylbenzene, and vinyltoluene; polymerizable monomers having a phenyl group and a sulfonate, such as styrene sulfonate, divinylbenzene sulfonate, o-methylstyrene sulfonate, and p-methylstyrene sulfonate; and polymerizable monomers having a naphthyl group, such as 1-vinylnaphthalene, 2-vinylnaphthalene, α-naphthyl (meth)acrylate, and β-naphthyl (meth)acrylate; for example, polymerizable unsaturated carboxylic acids such as (meth)acrylic acid, itaconic acid, maleic acid, and fumaric acid; for example, polymerizable unsaturated carboxylic acid esters such as methyl (meth)acrylate, ethyl (meth)acrylate, n-butyl (meth)acrylate, 2-hydroxyethyl (meth)acrylate, glycidyl (meth)acrylate, ethylene glycol di(meth)acrylic acid ester, and tribromophenyl (meth)acrylate; unsaturated carboxylic acid amides such as (meth)acrylonitrile, (meth)acrolein, (meth)acrylamide, N-methylol-(meth)acrylamide, methylenebis(meth)acrylamide, butadiene, isoprene, vinyl acetate, vinylpyridine, N-vinylpyrrolidone, vinyl chloride, vinylidene chloride, and vinyl bromide; polymerizable unsaturated nitriles; vinyl halides; and conjugated dienes.

The average particle size of the latex particles used in the LTIA can be selected from, for example, 0.02 to 1.6 μm, and preferably 0.1 μm to 0.4 μm, in consideration of the concentration of the measurement target substance in the sample, the detection sensitivity of the measuring instrument, or the like. Here, the average particle size can be measured using a particle size distribution meter.

The method of causing the latex particles used in the LTIA to carry one or more of the above-described specific affinity substances is not particularly limited, and the specific affinity substances may be caused to be carried by a known method such as physical adsorption (hydrophobic bonding) or chemical bonding.

For the purpose of preventing non-specific adsorption of impurities onto the latex particles carrying a specific affinity substance, the latex particles may be subjected to a known blocking treatment (masking treatment) of bringing the latex particles into contact with a protein such as bovine serum albumin (BSA), casein, gelatin, egg white albumin, or a salt thereof, a polymer compound such as a polysaccharide, a surfactant, or skimmed milk powder.

<Configuration of reagent>

The configuration of the immunological measurement reagent of the present aspect may be a configuration including the non-specific reaction inhibitor of the first aspect, and examples thereof include a two-reagent type configuration in which a first reagent contains the non-specific reaction inhibitor and a second reagent contains latex particles. In addition, the reagent may be of a multi-reagent type including a third or subsequent n-th reagent (n represents an integer of 3 or more) containing other components. The non-specific reaction inhibitor may be contained in the second reagent or the n-th reagent other than the first reagent, together with other components. It is preferable that the non-specific reaction inhibitor be contained in the first reagent.

The immunological measurement reagent of the present aspect may contain a buffer, a protein, a peptide, an amino acid, a nucleic acid, a lipid, a phospholipid, a sugar, an inorganic salt, a polymer compound, a surfactant, other non-specific reaction inhibitors, a preservative, and the like, which are known in the related art, to the extent that does not interfere with the non-specific reaction inhibitory effect. As a component that buffers and adjusts the pH, the ionic strength, the osmotic pressure, and the like of the sample, for example, a buffer solution of acetic acid, citric acid, phosphoric acid, Tris, glycine, boric acid, carbonic acid, phthalic acid, succinic acid, maleic acid, imidazole, or the like, a Good's buffer solution, or a sodium salt, a potassium salt, a calcium salt, or the like of those acids may be contained. Furthermore, a polymer such as polyvinylpyrrolidone or a phospholipid polymer, which is known in the related art, may be contained as a component that enhances the formation of agglutinates. Examples of the phospholipid polymer that enhances the formation of agglutinates include Lipidure (registered trademark) BL400 series, which is sold by NOF

CORPORATION.

As a specific example of the reagent configuration, as will be described in Examples described later, a two-reagent type immunological measurement reagent in which a reagent obtained by adding a non-specific reaction inhibitor to a buffer solution intended for diluting a sample is used as a first reagent, and a reagent obtained by dispersing latex particles carrying a specific affinity substance in the same buffer solution is used as a second reagent, may be mentioned.

Furthermore, in a case where a diluent, a pretreatment solution, or the like is added to a sample before the sample is subjected to an immunological measurement, the non-specific reaction inhibitor of the first aspect may be contained in the diluent or the pretreatment solution.

<Concentration of non-specific inhibitory substance>

Regarding the immunological measurement reagent of the present aspect, in a case where the first reagent contains the non-specific reaction inhibitor, the concentration of the non-specific reaction inhibitor in the first reagent is, for example, preferably 0.001 % to 5.00% (w/v), more preferably 0.002% to 1.00% (w/v), even more preferably 0.003% to 0.60% (w/v), particularly preferably 0.005% to 0.50% (w/v), and most preferably 0.04% to 0.30% (w/v) or 0.02% to 0.20% (w/v). When the concentration is within these suitable ranges, the dispersibility of the non-specific reaction inhibitor in the reagent is increased, and the non-specific reaction inhibitor is likely to be uniformly mixed in the sample.

<Method of using reagent>

As an example of a method of using the immunological measurement reagent according to the present aspect, the following methods (1) to (4) of bringing a biological sample containing a measurement target substance into contact with latex particles carrying a specific affinity substance for the measurement target substance in the presence of the non-specific reaction inhibitor of the first aspect, may be mentioned.

(1) A method of mixing a biological sample with a first reagent containing a non-specific reaction inhibitor, a buffer solution containing an optional component, and the like, and then mixing the mixed liquid with a second reagent containing latex particles that carry a specific affinity substance for a measurement target substance, a buffer solution containing an optional component, and the like.
(2) A method of simultaneously mixing a biological sample, a first reagent containing a non-specific reaction inhibitor, a buffer solution containing an optional component, and the like, and a second reagent containing latex particles that carry a specific affinity substance for a measurement target substance, a buffer solution containing an optional component, and the like.
(3) A method of mixing a biological sample with a second reagent containing latex particles that carry a specific affinity substance for a measurement target substance, a buffer solution containing optional components, and the like, and then adding a first reagent containing a non-specific reaction inhibitor, a buffer solution containing optional components, and the like to the mixed liquid to be mixed.

(4) A method of mixing a first reagent containing a non-specific reaction inhibitor, a buffer solution of an optional component, and the like, with a second reagent containing latex particles that carry a specific affinity substance for a measurement target substance, a buffer solution of an optional component, and the like, and then adding a biological sample to this mixed liquid to be mixed. Among the above-described methods, from the viewpoint of inhibiting a non-specific reaction, (1), (2), and (4) are preferred, (1) and (4) are more preferred, and (1) is most preferred.

Examples of a specific reagent of the present aspect include an sIL-2R measurement reagent used for an examination in which sIL-2R in a blood sample is measured by optically measuring or visually observing the degree of agglutination caused by an antigen-antibody reaction between sIL-2R in the blood sample and an insoluble carrier carrying an anti-sIL-2R antibody, the sIL-2R measurement reagent containing an insoluble carrier carrying an anti-sIL-2R antibody and the non-specific reaction inhibitor of the first aspect.
Examples of another specific reagent of the present aspect include an SP-D measurement reagent used for an examination in which SP-D in a blood sample is measured by optically measuring or visually observing the degree of agglutination caused by an antigen-antibody reaction between SP-D in the blood sample and an insoluble carrier carrying an anti-SP-D antibody, the SP-D measurement reagent containing an insoluble carrier carrying an anti-SP-D antibody and the non-specific reaction inhibitor of the first aspect.

<<Biochemical measurement reagent kit>>

A fifth aspect of the present invention is a reagent kit including the biochemical measurement reagent of the fourth aspect. Since the description of the first to fourth aspects can also be applied to the fifth aspect, any overlapping description will not be repeated.
Examples of the configuration other than the biochemical measurement reagent of the fourth aspect, which may be included in the present aspect, include a reagent instruction manual, a blood sample collecting tool (a collecting pipette, a syringe, a cotton swab, a filtration filter, and the like), a detecting apparatus, and an analyzing apparatus.

<<Specimen pretreatment solution>>

A sixth aspect of the present invention is a reagent used for biochemical measurement, the above-described reagent being a specimen pretreatment solution containing a copolymer that has a constitutional unit A derived from 2-methacryloyloxyethyl phosphorylcholine and exhibits the above-described physical property X, and a liquid medium in which the above-described copolymer is dissolved or dispersed.
Here, the biological sample, which is the specimen, may be human-derived or animal-derived. The sample may be blood, or may be a culture supernatant, urine, feces, cerebrospinal fluid, saliva, sweat, ascites, an extract of cells or tissues, or the like. The blood may be whole blood, may be plasma, may be serum, or may be a sample obtained by subjecting blood to any treatment such as dilution or purification.
Here, the specimen pretreatment is to add a certain component in advance to a specimen before mixing the specimen with a measurement reagent. For example, a solution containing the non-specific reaction inhibitor of the present invention can be added to the specimen, and before mixing the solution with the measurement reagent, the reaction between the non-specific factor in the specimen and the non-specific reaction inhibitor can be completed. A "specimen diluent" may be added for the purpose of simply diluting the specimen, and such a "specimen diluent" is also included in the "specimen pretreatment solution" of the present invention.
The above-described liquid medium is not particularly limited as long as it is a medium that is miscible with the measurement reagent, and it is preferably a water-based medium.
The specimen pretreatment solution may contain a buffer, a protein, a peptide, an amino acid, a nucleic acid, a lipid, a phospholipid, a sugar, an inorganic salt, a polymer compound, a surfactant, a non-specific reaction inhibitor that does not fall within the scope of the present invention, an antiseptic agent, or the like, which are known in the related art, to the extent that does not interfere with the non-specific reaction inhibitory effect of the non-specific reaction inhibitor of the present invention. As a component that buffers and adjusts the pH, the ionic strength, the osmotic pressure, and the like of a sample, which is a specimen, for example, a buffer solution of acetic acid, citric acid, phosphoric acid, Tris, glycine, boric acid, carbonic acid, phthalic acid, succinic acid, maleic acid, imidazole, or the like, a Good's buffer solution, or a sodium salt, a potassium salt, a calcium salt, or the like of those acids may be contained. Furthermore, particularly in the case of the LTIA reagent, a polymer such as polyvinylpyrrolidone or a phospholipid polymer, which is known in the related art, may be contained as a component that enhances the formation of agglutinates of a solute (nonvolatile component) to be mixed with the specimen pretreatment solution. Examples of the phospholipid polymer that enhances the formation of agglutinates include Lipidure (registered trademark) BL400 series, which is sold by NOF CORPORATION.
In a case where a 0.1-fold to 10-fold volume of the specimen pretreatment solution is added to a sample, which is a specimen, the concentration of the above-described copolymer with respect to the total volume of the specimen

pretreatment solution is, for example, preferably 0.001% to 5.00% (w/v), more preferably 0.01% to 4.50% (w/v), and even more preferably 0.10% to 3.00% (w/v), 0.20% to 2.50% (w/v), or 0.5% to 2.00% (w/v). When the concentration is in these suitable ranges, the dispersibility of the non-specific reaction inhibitor in the specimen pretreatment solution is increased, and the non-specific reaction inhibitor is likely to be mixed uniformly with the sample specimen.

The specimen pretreatment solution can be used by being mixed with a specimen at any ratio. For example, 0.1 to 10 volumes of the specimen pretreatment solution can be added to 1 volume of the specimen and used. When mixing the specimen with the specimen pretreatment solution, the mixing may be performed by a manual method using a micropipette or the like, or may be automatically performed using a specimen diluting apparatus. After the specimen is mixed with the specimen pretreatment solution, the measurement may be carried out after incubation at 10°C to 40°C for 30 seconds to 60 minutes or the like is carried out.

Examples

[Preliminary Test 1] Measurement of infrared absorption spectrum

1. Measurement method

500 μL of each Lipidure (5 wt% aqueous solution) shown in Table 1 was added to 5,000 μL of tetrahydrofuran (THF), and the components of Lipidure were precipitated. Subsequently, centrifugation was carried out at 8000 rpm for 3 minutes to remove the supernatant. 5000 μL of THF was added again to the obtained precipitates, the components of Lipidure were precipitated, and centrifugation was carried out again at 8000 rpm for 3 minutes. The supernatant was removed, and the residue was dried at 95°C for 7 hours. An infrared absorption spectrum of the obtained Lipidure powder was measured by Fourier transform infrared spectroscopy.

The results are shown in FIG. 1A to FIG. 1H. The legends in the figures represent the model numbers following "Lipidure-" in Table 1.

<Measuring apparatus>

Nicolet™ iS5 FT-IR manufactured by Thermo Fisher Scientific, Inc.

<Measurement conditions>

Crystal: Diamond
Angle of incidence: 45°
Correction: None

2. Measurement results

Table 1 shows the measurement results of the infrared absorption spectrum, and the side chain properties described on the NOF Corporation website, the physical property data of the kinematic viscosity and the surface tension which are read from the figure described in the catalog.

In the table, in the column of "Constitutional unit B has no functional group that ionizes", "O" is described in a case where the constitutional unit B does not have a functional group that ionizes, and "X" is described in a case where the constitutional unit B has a functional group that ionizes.

In the column of "P1: There is a maximum absorption at 2946 to 2906 $cm^{-1}$", "O" is described in a case where there is a maximum absorption, and "X" is described in a case where there is no maximum absorption. The maximum absorption was present at 2924 $cm^{-1}$ in Lipidure-BL1002 and Lipidure-BL1003, and at 2926 $cm^{-1}$ in Lipidure-BL1103.

In the column of "P2: There is a maximum absorption at 2870 to 2833 $cm^{-1}$", "O" is described in a case where there is a maximum absorption, and "X" is described in a case where there is no maximum absorption. The maximum absorption was present at 2852 $cm^{-1}$ in Lipidure-BL1002 and Lipidure-BL1003, and at 2854 $cm^{-1}$ in Lipidure-BL1103.

The transmittance (%) of P1 and the transmittance (%) of P2 were such that P1 < P2 in all of Lipidure-BL1002, 1003, and 1103.

P3 is a peak having a maximum absorption in the range of 975 to 940 $cm^{-1}$. All the measured samples had a maximum absorption at P3. The wavenumber varied depending on the model number of Lipidure, and was in the range of 964 to 951 $cm^{-1}$.

P4 is a peak having an absorption maximum in the range of 1252 to 1215 $cm^{-1}$. The measured samples, except for

Lipidure-BL405, had a maximum absorption at P4. The wavenumber varied depending on the model number of Lipidure, and was in the range of 1242 to 1225 $cm^{-1}$.

In the table, the columns of Ratio A to Ratio D describe the following. The intensity and the ratio were calculated by the methods of Formula (1) and Formula (2) in the specification.

Ratio A: P1/P3 was calculated in a case where P1 was present. In a case where P1 was not present, the ratio of the intensity at 2925 $cm^{-1}$ to the intensity of P3 was calculated for model numbers whose side chain property was "hydrophobic" in Table 1B.

Ratio B: P1/P4 was calculated in a case where P1 was present. In a case where P1 was not present, the ratio of the intensity at 2925 $cm^{-1}$ to the intensity of P4 was calculated for model numbers whose side chain property was "hydrophobic" in Table 1B.

Ratio C: P2/P3 was calculated in a case where P2 was present. In a case where P2 was not present, the ratio of the intensity at 2852 $cm^{-1}$ to the intensity of P3 was calculated for model numbers whose side chain property was "hydrophobic" in Table 1B.

Ratio D: P2/P4 was calculated in a case where P2 was present. In a case where P2 was not present, the ratio of the intensity of 2852 $cm^{-1}$ to the intensity of P4 was calculated for model numbers whose side chain property was "hydrophobic" in Table 1B.

In the table, "N.D." means that the measurement was not performed, the calculation was not possible due to the absence of P1 or P2, or there was no information on the website or the catalog.

[Table 1A]

| Lipidure BL series Model number | IR spectrum | | | | | |
|---|---|---|---|---|---|---|
| | P1: There is maximum absorption at 2946 to 2906$cm^{-1}$ | P2: There is maximum absorption at 2870 to 2833$cm^{-1}$ | Ratio A | Ratio B | Ratio C | Ratio D |
| Lipidure-NH01 | X | X | N.D. | N.D. | N.D. | N.D. |
| Lipidure-AC01 | X | X | N.D. | N.D. | N.D. | N.D. |
| Lipidure-SF08 | N.D. | N.D. | N.D. | N.D. | N.D. | N.D. |
| Lipidure-BL103 | X | X | N.D. | N.D. | N.D. | N.D. |
| Lipidure-BL203 | X | X | 13.8% | 15.8% | 4.5% | 5.2% |
| Lipidure-BL206 | O | X | 26.5% | 28.3% | 6.5% | 6.9% |
| Lipidure-BL405 | X | X | N.D. | N.D. | N.D. | N.D. |
| Lipidure-BL502 | X | X | N.D. | N.D. | N.D. | N.D. |
| Lipidure-BL802 | X | X | N.D. | N.D. | N.D. | N.D. |
| Lipidure-BL803 | X | X | N.D. | N.D. | N.D. | N.D. |
| Lipidure-BL804 | X | X | N.D. | N.D. | N.D. | N.D. |
| Lipidure-BL1201 | X | X | 15.0% | 16.7% | 7.7% | 8.6% |
| Lipidure-BL1202 | X | X | 14.4% | 16.9% | 6.6% | 7.8% |
| Lipidure-BL1301 | X | X | 15.1% | 17.1% | 6.9% | 7.8% |
| Lipidure-BL1002 | O | O | 38.6% | 44.1% | 28.8% | 32.9% |
| Lipidure-BL1003 | O | O | 44.1% | 49.4% | 28.1% | 31.4% |
| Lipidure-BL1103 | O | O | 28.8% | 32.3% | 19.5% | 21.9% |

[Table 1B]

| Lipidure BL series Model number | Information described in manufacturer's website or catalog | | | | | |
|---|---|---|---|---|---|---|
| | Comprising constitutional unit A | Being copolymer | Side chain property | Constitutional unit B has no functional group that ionizes | Kinematic viscosity (5 wt%, 25°C) is within range of 0.2 to 4.0 [$m^2/s$] | Surface tension (0.1 wt%, 25°C) is within range of 45 to 70 [$\times 10^{-3}$ N/m] |
| Lipidure-NH01 | O | O | Having amino group | X | N.D. | N.D. |
| Lipidure-AC01 | O | O | Having carboxyl group | X | N.D. | N.D. |
| Lipidure-SF08 | O | X | N.D. | N.D. | N.D. | N.D. |
| Lipidure-BL103 | O | O | Hydrophilic | X | X | X |
| Lipidure-BL203 | O | O | Hydrophobic | O | X | O |
| Lipidure-BL206 | O | O | Hydrophobic | O | O | X |
| Lipidure-BL405 | O | O | Anionic | X | X | X |
| Lipidure-BL502 | O | O | Cationic | X | X | X |
| Lipidure-BL802 | O | O | Hydrophilic/hydrophobic | X | O | O |
| Lipidure-BL803 | O | O | Hydrophilic/hydrophobic | X | O | O |
| Lipidure-BL804 | O | O | Hydrophilic/hydrophobic | X | O | O |
| Lipidure-BL1201 | O | O | Hydrophobic | O | X | O |
| Lipidure-BL1202 | O | O | Hydrophobic | O | O | O |
| Lipidure-BL1301 | O | O | Hydrophobic | O | X | O |
| Lipidure-BL1002 | O | O | Hydrophobic | O | O | O |
| Lipidure-BL1003 | O | O | Hydrophobic | O | O | O |
| Lipidure-BL1103 | O | O | Hydrophobic | O | O | O |

[Experimental Example 1] Use of non-specific reaction inhibitor in soluble interleukin 2 receptor (sIL-2R) measurement reagent - 1

The following describes a test in which the concentration of sIL-2R in a sample was measured using a reagent according to the LTIA method, to which Lipidure-BL1002, 1003, and 1103, which are examples of the non-specific reaction inhibitor of the present invention, and other components were added. The following materials were used.

· HBR-1 manufactured by SCANTIBODIES LABORATORY, INC. was used.
· Various Lipidures manufactured by NOF CORPORATION were used.

1. Measurement by CLEIA method (Reference Example 1)

A CLEIA method performs a B/F separation operation and has a washing step. For this reason, this is a measurement method that is difficult to be affected by a non-specific reaction caused by a sample. The measured value obtained by the present method was used as a control value for the sIL-2R concentration in each specimen.

1-1. Measurement reagent

Lumipulse Presto (registered trademark) IL-2R (Fujirebio Inc.)

1-2. Sample

Human serum specimen Specimen numbers 1 to 17 (however, the donor of each specimen is different.)

1-3. Measurement procedure

Measurement was carried out according to the package insert of the measurement reagent using LUMIPULSE (registered trademark) -L2400 (Fujirebio Inc.).

2. Measurement using LTIA method reagent (Comparative Examples 1 to 16 and Examples 1 to 3)

2-1. Measurement reagent

A first reagent and a second reagent were prepared according to the methods described in Japanese Unexamined Patent Application, First Publication No. 2017-181377 and Japanese Unexamined Patent Application, First Publication No. 2018-173429.

· In Comparative Example 1, the above-described first reagent was used as it was.
· In Comparative Example 2, a reagent obtained by adding HBR-1, a commercially available non-specific reaction inhibitor, to the above-described first reagent to a concentration of 100 μg/mL was used.
· In Comparative Examples 3 to 16 and Examples 1 to 3, reagents obtained by adding various Lipidures shown in Table 2 to the above-described first reagent to a final concentration of 0.1 % (w/v) were used.

[Table 2]

| | Measurement method | Additive | Concentration |
|---|---|---|---|
| Reference Example 1 | CLEIA method | - | - |
| Comparative Example 1 | LTIA method | No additive | - |
| Comparative Example 2 | | HBR-1 | 100 μg/mL |
| Comparative Example 3 | | Lipidure-NH01 | 0.10% |
| Comparative Example 4 | | Lipidure-AC01 | |
| Comparative Example 5 | | Lipidure-SF08 | |
| Comparative Example 6 | | Lipidure-BL103 | |
| Comparative Example 7 | | Lipidure-BL203 | |
| Comparative Example 8 | | Lipidure-BL206 | |
| Comparative Example 9 | | Lipidure-BL405 | |
| Comparative Example 10 | | Lipidure-BL502 | |
| Comparative Example 11 | | Lipidure-BL802 | |
| Comparative Example 12 | | Lipidure-BL803 | |
| Comparative Example 13 | | Lipidure-BL804 | |
| Comparative Example 14 | | Lipidure-BL1201 | |
| Comparative Example 15 | | Lipidure-BL1202 | |
| Comparative Example 16 | | Lipidure-BL1301 | |
| Example 1 | | Lipidure-BL1002 | |
| Example 2 | | Lipidure-BL1003 | |
| Example 3 | | Lipidure-BL1103 | |

2-2. Sample

The same as the samples of 1-2.

2-3. Measurement procedure

The first reagent and the second reagent were combined, and the sIL-2R concentration in a sample was measured using a Hitachi 7180 automatic analyzing apparatus. Specifically, 120 μL of the first reagent was added to 5.6 μL of the sample, the mixture was kept warm at 37°C for 5 minutes, 40 μL of the second reagent was added thereto, and the mixture was stirred. The change in absorbance associated with the agglutinate formation was measured at a main wavelength of 570 nm and a subwavelength of 800 nm for 5 minutes, and the amount of change in absorbance was applied to a calibration curve obtained by measuring a standard substance with known concentrations to calculate a measured value. For each specimen, a value obtained by dividing the measured value in each of Comparative Examples 1 to 16 and Examples 1 to 3 by the measured value of Reference Example 1 was calculated and evaluated as a relative ratio (%). In a case where the relative ratio was 85% or more and 115% or less, it was determined that the measured value was not deviated from the measured value of Reference Example 1.

3. Results and consideration

Measurement results are shown in Table 3. In the table, "N.D." indicates that the measurement was not carried out.

The measurement sensitivity of a calibration curve obtained by measuring a standard substance with known concentrations (net measurement sensitivity due to an antigen-antibody reaction obtained by subtracting the measurement sensitivity of a physiological saline under each condition) was calculated. In a case where the sensitivity of Comparative Example 1 was set to 100%, the sensitivity decreased to 86% in Comparative Example 3. Furthermore, in Comparative Example 9, the sensitivity increased to 115%. Under other conditions, the sensitivity was approximately the same, such as 101 % to 111 %.

[Table 3]

| Specimen No. | sIL-2R measured value Relative ratio (%) with respect to Reference Example 1 | | | | | | | | | | | | | | | | |
|---|---|---|---|---|---|---|---|---|---|---|---|---|---|---|---|---|---|
| | Control specimen | | | | | | | Deviation specimen | | | | | | | | | |
| | 1 | 2 | 3 | 4 | 5 | 6 | 7 | 8 | 9 | 10 | 11 | 12 | 13 | 14 | 15 | 16 | 17 |
| Reference Example 1 | 100% | 100% | 100% | 100% | 100% | 100% | 100% | 100% | 100% | 100% | 100% | 100% | 100% | 100% | 100% | 100% | 100% |
| Comparative Example 1 | 102% | 107% | 103% | 111% | 104% | 104% | 107% | 124% | 115% | 162% | 119% | 149% | 137% | 121% | 122% | 124% | 127% |
| Comparative Example 2 | 107% | 107% | 96% | 111% | 107% | 103% | 109% | 126% | 126% | 160% | 118% | 147% | 141% | 122% | 121% | 123% | 126% |
| Comparative Example 3 | 108% | 111% | 109% | 116% | 110% | 111% | 109% | 129% | 108% | 178% | 128% | 158% | 147% | 127% | 125% | 127% | 129% |
| Comparative Example 4 | 110% | 116% | 110% | 111% | 104% | 108% | 109% | 130% | 111% | 176% | 127% | 156% | 147% | 126% | 126% | 128% | 129% |
| Comparative Example 5 | 134% | 141% | 125% | 138% | 118% | 123% | 119% | 171% | 153% | 589% | 192% | 299% | 191% | 168% | 167% | 151% | 167% |
| Comparative Example 6 | 107% | 114% | 110% | 118% | 110% | 113% | 106% | 130% | 104% | 201% | 141% | 162% | 151% | 127% | 126% | 127% | 127% |
| Comparative Example 7 | 111% | 119% | 115% | 124% | 116% | 117% | 109% | 134% | 119% | 208% | 144% | 168% | 157% | 131% | 132% | 131% | 134% |
| Comparative Example 8 | 108% | 104% | 107% | 107% | 104% | 106% | 109% | 117% | 108% | 147% | 110% | 130% | 124% | 113% | 115% | 116% | 112% |
| Comparative Example 9 | 83% | 83% | 98% | 108% | 101% | 101% | 99% | 134% | 72% | 247% | 152% | 171% | 164% | 128% | 129% | 127% | 131% |
| Comparative Example 10 | 99% | 110% | 107% | 119% | 111% | 114% | 106% | 130% | 114% | 196% | 137% | 163% | 151% | 128% | 127% | 125% | 128% |
| Comparative Example 11 | 111% | 112% | 107% | 113% | 106% | 108% | 108% | 127% | 119% | 177% | 122% | 148% | 137% | 122% | 123% | 122% | 122% |
| Comparative Example 12 | 116% | 112% | 113% | 117% | 107% | 110% | 110% | 129% | 122% | 180% | 125% | 153% | 143% | 125% | 126% | 126% | 128% |
| Comparative Example 13 | 113% | 121% | 109% | 114% | 111% | 111% | 113% | 133% | 126% | 179% | 127% | 159% | 148% | 128% | 130% | 131% | 132% |
| Comparative Example 14 | 109% | 111% | 112% | 116% | 109% | 108% | 108% | 129% | 122% | 183% | 132% | 161% | 149% | 127% | 128% | 127% | 127% |
| Comparative Example 15 | 109% | 106% | N.D. | 107% | 110% | 106% | 113% | 103% | 163% | 121% | 146% | 143% | 123% | 130% | 126% | 121% | N.D. |
| Comparative Example 16 | 113% | 111% | 110% | 117% | 112% | 113% | 107% | 129% | 127% | 192% | 135% | 160% | 148% | 127% | 126% | 126% | 127% |
| Example 1 | 99% | 109% | 104% | 105% | 108% | 106% | 106% | 103% | 97% | 113% | 97% | 107% | 111% | 103% | 100% | 107% | 99% |
| Example 2 | 105% | 109% | 109% | 113% | 112% | 109% | 111% | 106% | 114% | 111% | 99% | 106% | 111% | 105% | 104% | 112% | 100% |
| Example 3 | 93% | 89% | 96% | 98% | 99% | 92% | 100% | 109% | 108% | N.D. | 101% | N.D. | N.D. | 103% | 101% | 108% | N.D. |

(1) Control specimen

Specimens 1 to 7 in which the proportion (relative ratio) of the measured value of the LTIA method reagent to which no additive was added (Comparative Example 1), with respect to the measured value of the CLEIA method (Reference Example 1) was 102% to 111 %, were evaluated as "control specimens" in which a non-specific reaction did not occur in the LTIA method. In the control specimens, the measured values (Examples 1 to 3) of the LTIA method reagent to which Lipidure-BL1002, Lipidure-BL1003, and Lipidure-BL1103 of the present invention were added showed relative ratios of 89% to 113% with respect to Reference Example 1, and measurement results roughly equivalent to those of Reference Example 1 were obtained. Therefore, it was found that the addition of Lipidure-BL1002, Lipidure-BL1003, and Lipidure-BL1103 of the present invention did not affect the measured value of a specimen that does not exhibit a non-specific reaction.

In Comparative Example 3, the relative ratio of the specimen 4 was 116%, which deviated from the range of 85% or more and 115% or less, and the relative ratio was poor as compared with the condition in which the present compound was not added (Comparative Example 1). In addition, the relative ratios of the specimen 2 in Comparative Example 4, all of the specimens 1 to 7 in Comparative Example 5, and the specimen 4 in Comparative Example 6 deviated from the range of 85% or more and 115% or less, so that even in the control specimens in the Comparative Examples, there were many cases where the relative ratio was poor due to the addition of Lipidure that does not have the properties of the present invention. All the control specimens could be measured with relative ratios similar to those of Reference Example 1 and

Comparative Example 1 only in Comparative Examples 2, 8, 11, and 15 and Examples 1, 2, and 3.

(2) Measured value of deviation specimen

Specimens 8 to 17 in which the proportion (relative ratio) of the measured value of the LTIA method reagent to which no additive was added (Comparative Example 1) with respect to the measured value of the CLEIA method (Reference Example 1) was less than 85% or greater than 115%, were evaluated as "deviation specimens" that cause a non-specific reaction in the LTIA method.

In Specimen number 8, the relative ratio of the measured value of the LTIA method reagent to which the non-specific reaction inhibitor was not added (Comparative Example 1) was 124%, which deviated from that of Reference Example 1, and it was considered that a non-specific reaction had occurred. In this regard, in a case where HBR-1, which is a commercially available non-specific reaction inhibitor, was added (Comparative Example 2), the relative ratio was 126%, and the deviation in Specimen number 8 was not improved by HBR-1. In a case where 0.1 % of Lipidure that does not have the property of the present invention was added (Comparative Examples 3 to 14 and 16), the relative ratio was 117% to 171%, and the measured values deviated further. On the other hand, in the measured values (Examples 1 to 3) obtained using the LTIA method reagent to which 0.1 % (w/v) of Lipidure-BL1002, Lipidure-BL1003, and Lipidure-BL1103 having the property of the present invention were added, the relative ratios were 103%, 106%, and 109%, and the relative ratios with respect to Reference Example 1 were improved. Similar results were also obtained in the specimens 9 to 17. In Comparative Example 15, the relative ratio was improved in Specimen number 8; however, the relative ratio deviated greatly in the specimens 9 to 17.

As described above, in the LTIA method which is a homogeneous immunoassay method, a non-specific reaction occurring due to the sample could be inhibited by allowing Lipidure-BL1002, Lipidure-BL1003, and Lipidure-BL1103 having the property of the present invention to be present in the reaction system. All of the Lipidure series are high-molecular-weight polymers comprising a segment derived from 2-methacryloyloxyethyl phosphorylcholine; however, when the Lipidure series were added to the LTIA reagent, many specimens having a poor relative ratio occurred even in the control specimens in which the relative ratio was measured to be 85% or more and 115% or less with respect to Reference Example 1 when the Lipidure series were not added. The only components that exhibited an effect of inhibiting the non-specific reaction, which has been a problem so far, and reducing the deviation of the measured value without affecting the measured value of the control specimens, were Lipidure-BL1002, 1003, and 1103, which had been found by the present inventors.

It is noted that Lipidure-NH01 of Comparative Example 3 does not satisfy the physical property X related to the infrared absorption spectrum, and thus does not fall within the scope of the present invention. Furthermore, Lipidure-NH01 is a reactive polymer having an amino group, and can react with a functional group (a carboxyl group or the like) on the surface of a base material. Therefore, it is considered that Lipidure-NH01 is not preferable from the viewpoint of having an amino group, in addition to the viewpoint that the polymer does not satisfy the above-described physical property X.

Lipidure-AC01 of Comparative Example 4 does not satisfy the above-described physical property X related to the infrared absorption spectrum, and does not fall within the scope of the present invention. Furthermore, the polymer is a copolymer of a constitutional unit A derived from 2-methacryloyloxyethyl phosphorylcholine and a constitutional unit having a carboxyl group in a side chain. That is, the constitutional unit has a functional group that ionizes in water. Therefore, it is considered that Lipidure-AC01 is not preferable from the viewpoint of comprising a constitutional unit having a functional group that ionizes in water, in addition to the viewpoint that the polymer does not satisfy the above-described physical property X.

Lipidure-SF08 of Comparative Example 5 is a substance having a phosphorylcholine group, and is sold as a low-molecular-weight amphoteric surfactant. The polymer is not a copolymer and does not fall within the scope of the present invention.

According to the catalog, it is known that Lipidure has an effect of improving sensitivity (Lipidure-BL103, 400 series), a non-specific adsorption inhibitory effect (blocking) (Lipidure-BL200, 800, 1200 series), and an effect of stabilization (Lipidure-BL800 series, SF08) in a latex immunoturbidimetric system; however, it is not known that there is an effect of inhibiting deviation of a measured value for a specimen exhibiting a non-specific reaction. The present invention has found a new, heretofore unknown effect of inhibiting non-specific reactions in the Lipidure-BL1000 series and the Lipidure-BL1100 series, which is different from the effects previously known.

It has been found that the copolymer of the present aspect can inhibit non-specific reactions attributed to lipids and immunoglobulins by a method known in the related art. By verifying the change in the measured value when components that may be non-specific factors are added to the sample or when these components are removed, it is possible to estimate which components are non-specific factors. A possibility that the copolymer of the present aspect may inhibit a non-specific reaction caused by a non-specific factor other than the above-described ones, may also be considered.

According to the catalog, Lipidure-BL405 studied in Comparative Example 9 is presumed to be the component

described in Patent Document 7, which is said to be "highly hydrophilic" and to exhibit an effect of inhibiting serum interference. However, this component did not exhibit an effect of reducing deviation of the measured value, and even the measurement accuracy of the control specimen was poor.

[Experimental Example 2] Use of non-specific reaction inhibitor in soluble interleukin 2 receptor (sIL-2R) measurement reagent - 2

1. Measurement using LTIA method reagent

1-1. Measurement reagent

A first reagent and a second reagent were prepared according to the methods described in Japanese Unexamined Patent Application, First Publication No. 2017-181377 and Japanese Unexamined Patent Application, First Publication No. 2018-173429. Lipidure-BL1002 found in the present application was added to the first reagent to have the following final concentration.

· Example 4 0.01% (w/v)
· Example 5 0.05% (w/v)
· Example 6 0.2% (w/v)
· Example 7 0.5% (w/v)

1-2. Sample

Human serum specimen Specimen numbers 8, 11, 13, and 15 used in Experimental Example 1

1-3. Measurement procedure

The same as that in 2-3 of Experimental Example 1

2. Results and consideration

Measurement results are shown in Table 4.
The measurement sensitivity of a calibration curve obtained by measuring a standard substance with known concentrations (net measurement sensitivity due to an antigen-antibody reaction obtained by subtracting the measurement sensitivity of a physiological saline under each condition) was calculated. In a case where the sensitivity of Comparative Example 1 was set to 100%, the measurement sensitivities of Examples 1 and 4 to 6 were 100% to 108%, which were roughly equivalent to that of Comparative Example 1. The measurement sensitivity of Example 7 was 92%.

[Table 4]

| Specimen No. | Lipidure-BL1002 concentration % (w/v) | sIL-2R measured value Relative ratio (%) with respect to Reference Example 1 | | | |
|---|---|---|---|---|---|
| | | Deviation specimen | | | |
| | | 8 | 11 | 12 | 15 |
| Reference Example 1 | - | 100% | 100% | 100% | 100% |
| Comparative Example 1 | 0% | 124% | 119% | 149% | 122% |
| Example 1 | 0.1% | 103% | 97% | 107% | 100% |
| Example 4 | 0.005% | 118% | 114% | 135% | 115% |
| Example 5 | 0.05% | 102% | 98% | 111% | 100% |
| Example 6 | 0.2% | 109% | 103% | 109% | 105% |
| Example 7 | 0.5% | 123% | 115% | 136% | 119% |

The concentration of Lipidure-BL1002 to be added to the first reagent was changed between 0.005% (w/v) and

0.5% (w/v). In all of the Specimen numbers 8, 11, 12, and 15, the relative ratio was improved as compared with Comparative Example 1. In particular, 0.05% (w/v) to 0.2% (w/v) is a preferable condition in which the relative ratio is 85% or more and 115% or less for all the specimens, and it was shown that there is an effect of inhibiting a non-specific reaction.

[Experimental Example 3] Use of non-specific reaction inhibitor in soluble interleukin 2 receptor (sIL-2R) measurement reagent - 3

1. Measurement by CLEIA method (Reference Example 2)

1-1. Measurement reagent

Same as in Reference Example 1

1-2. Sample

Human serum specimen 85 specimens (however, the donor of each specimen is different.)

1-3. Measurement procedure

Same as in Reference Example 1

2. Measurement using LTIA method reagent

2-1. Measurement reagent

· In Comparative Example 16, the same reagent as that of Comparative Example 1 was used.
· In Example 8, the same reagent as that of Example 1 was used.

2-2. Sample

Same as in Reference Example 2

2-3. Measurement procedure

Same as in Experimental Example 1

3. Results and consideration

A scatter diagram (FIG. 2(a)) was created, with the measured value according to Reference Example 2 on the axis of abscissa and the measured value according to Comparative Example 16 on the axis of ordinate. Furthermore, a scatter diagram (FIG. 2(b)) was created, with the measured value according to Reference Example 2 on the axis of abscissa and the measured value according to Example 8 on the axis of ordinate. In addition, the correlation coefficient and the slope of the regression line calculated by the least squares method for each plot are shown in the diagrams.
As shown in FIG. 2(a), in Comparative Example 16, the slope of the regression equation for the measured value with respect to Reference Example 2 was 1.11, and the correlation coefficient R was 0.983. In this regard, as shown in FIG. 2(b), the slope was 0.97 and R = 0.992in Example 8. The LTIA reagent to which the component of the present invention was added showed a significant improvement in correlation convergence with respect to the measured value of the CLEIA method. In particular, the improvement was remarkable in the specimens distributed between 1,000 and 3,000 U/mL.

[Experimental Example 4] Use of non-specific reaction inhibitor in pulmonary surfactant protein D (SP-D) measurement reagent - 1

The following describes a test in which the SP-D concentration in a sample was measured using a reagent according to the LTIA method to which Lipidure-BL1002 and 1003, which are the non-specific reaction inhibitors of the present invention, and other components have been added. The following materials were used.

· HBR-1 manufactured by SCANTIBODIES LABORATORY, INC. was used.
· Various Lipidures manufactured by NOF CORPORATION were used.

1. Measurement by CLEIA method (Reference Example 3)

A CLEIA method performs a B/F separation operation and has a washing step. For this reason, this is a measurement method that is difficult to be affected by a non-specific reaction caused by a sample. The measured value obtained by the present method was used as a control value for the SP-D concentration in each specimen.

1-1. Measurement reagent

CL SP-D "YAMASA" NX (manufactured by YAMASA CORPORATION)

1-2. Sample

Human serum specimen Specimen numbers 18 to 22 (however, the donor of each specimen is different.)

1-3. Measurement procedure

The measurement was carried out according to the package insert of the above-described measurement reagent using a fully automated chemiluminescence immunoassay analyzing apparatus CL-JACK NX.

2. Measurement using LTIA method reagent

2-1. Measurement reagent (Comparative Example 17)

(1) First reagent

100 mM MES-NaOH (pH 6.0)
500 mM NaCl
0.5% (w/v) BSA
Sensitizer (not Lipidure-BL1002, 1003, or 1103)

(2) Second reagent

Anti-human SP-D monoclonal antibody-sensitized latex
5 mM MOPS-NaOH (pH 7.0)

The anti-human SP-D monoclonal antibody-sensitized latex was prepared as follows. An anti-human SP-D monoclonal antibody solution diluted to 0.35 mg/mL with an equal amount of a 10 mM MOPS buffer solution was added to a 1% (w/v) polystyrene latex solution (manufactured by Sekisui Medical Co., Ltd.) (10 mM MOPS buffer solution) with an average particle size of 317 nm, and the mixture was stirred at 4°C for 2 hours. Thereafter, an equal amount of a 0.5% (w/v) BSA solution (10 mM MOPS buffer solution) was added thereto, the mixture was stirred at 4°C for 1 hour, and an anti-human SP-D monoclonal antibody-sensitized latex solution was prepared.
The anti-human SP-D monoclonal antibody was acquired using human SP-D (manufactured by GenScript Biotech Corporation) as an antigen by a method well known to those skilled in the art.

2-2. LTIA reagent (Comparative Examples 18 to 22 and Examples 9 and 10)

The first reagent and the second reagent described in Comparative Example 17 were used. However, as the first reagent, a reagent in which various components shown in Table 5 were added to the first reagent of Comparative Example 17 to the final concentrations indicated in the table, was used.

[Table 5]

| | Additive | Concentration |
|---|---|---|
| Comparative Example 17 | No additive | - |
| Comparative Example 18 | HBR-1 | 100 μg/mL |
| Comparative Example 19 | Lipidure-BL206 | 0.10% |
| Comparative Example 20 | Lipidure-BL802 | |
| Comparative Example 21 | Lipidure-BL1201 | |
| Comparative Example 22 | Lipidure-BL1301 | |
| Example 9 | Lipidure-BL1002 | |
| Example 10 | Lipidure-BL1003 | |

2-3. Calibrator

- 100 mM citrate buffer solution (pH 5.0)
- 1% (w/v) BSA
- 10 mM calcium chloride
- SP-D antigen

2-4. Sample

Same as 1-2.

3. Measurement method

The first reagent and the second reagent of each of Examples and Comparative Examples were combined, and the sample was measured using a Hitachi automatic analyzing apparatus 3500. Specifically, 120 μL of the first reagent was added to 5 μL of each sample, and the mixture was kept warm at 37°C for 5 minutes. Then, 40 μL of the second reagent was added thereto and stirred. Subsequently, the change in absorbance for 5 minutes was measured at a main wavelength of 570 nm and a subwavelength of 800 nm.

4. Evaluation method

First, the calibrator was measured, and a calibration curve for each test using each reagent was created. Each specimen was measured using the obtained calibration curve, and the concentration was determined. In a case where the measurement sensitivity of Comparative Example 17 was set to 100%, the measurement sensitivity was roughly equivalent at 97% to 106% under each condition.

For each specimen, a value obtained by dividing the measured value in each of Comparative Examples 17 to 22 and Examples 9 and 10 by the measured value of Reference Example 3 was calculated and evaluated as a relative ratio (%). In a case where the relative ratio was 90% or more and 110% or less, it was determined that the measured value was not deviated from the measured value of Reference Example 3.

[Table 6]

| Specimen No. | SP-D measurement Relative ratio (%) | | | | |
|---|---|---|---|---|---|
| | Control specimen | | | Deviation specimen | |
| | 18 | 19 | 20 | 21 | 22 |
| Reference Example 3 | 100% | 100% | 100% | 100% | 100% |

(continued)

| Specimen No. | SP-D measurement Relative ratio (%) | | | | |
|---|---|---|---|---|---|
| | Control specimen | | | Deviation specimen | |
| | 18 | 19 | 20 | 21 | 22 |
| Comparative Example 17 | 94% | 109% | 92% | 86% | 87% |
| Comparative Example 18 | 97% | 92% | 93% | 90% | 90% |
| Comparative Example 19 | 95% | 101% | 94% | 88% | 88% |
| Comparative Example 20 | 93% | 105% | 92% | 87% | 87% |
| Comparative Example 21 | 100% | 88% | 93% | 87% | 86% |
| Comparative Example 22 | 93% | 95% | 92% | 86% | 86% |
| Example 9 | 100% | 109% | 99% | 90% | 94% |
| Example 10 | 103% | 104% | 100% | 95% | 96% |

5. Results and consideration

(1) Control specimen

Specimens 18 to 20 in which the relative ratio of the measured value of the LTIA method reagent to which no additive was added (Comparative Example 17), with respect to the measured value of the CLEIA method (Reference Example 3) was 92% to 109%, were evaluated as "control specimens" in which a non-specific reaction did not occur in the LTIA method. In the control specimens, the measured values (Examples 9 and 10) of the LTIA method reagent to which Lipidure-BL1002 and Lipidure-BL1003 of the present invention were added showed relative ratios of 99% to 109% with respect to Reference Example 3, and measurement results roughly equivalent to those of Comparative Example 17 were obtained. Therefore, it was found that the addition of Lipidure-BL1002 and Lipidure-BL1003 having the property of the present invention did not affect the measured value of a specimen that does not exhibit a non-specific reaction.

(2) Measured value of deviation specimen

Specimens 21 and 22 in which the relative ratio of the measured value of the LTIA method reagent to which no additive was added (Comparative Example 17), with respect to the measured value of the CLEIA method (Reference Example 3) was less than 90%, were evaluated as "deviation specimens" in which a non-specific reaction occurred in the LTIA method.

In Specimen number 21, the relative ratio of the measured value of the LTIA method reagent (Comparative Example 17) to which no additive for the CLEIA method was added was 86%. In this regard, in a case where 0.1 % (w/v) of Lipidure that does not have the property of the present invention was added (Comparative Examples 19 to 22), the relative ratio was 86% to 88%, and the relative ratio almost did not change. On the other hand, in the measured values (Examples 9 and 10) of the LTIA method reagent to which 0.1 % (w/v) of Lipidure-BL1002 and Lipidure-BL1003 having the property of the present invention were added, the relative ratios were 90% and 95%, and the relative ratios were improved. Since the relative ratio in a case where HBR-1, which is a commercially available non-specific reaction inhibitor, was added (Comparative Example 18) was 90%, Lipidure-BL1002 had an effect of improving the relative ratio similar to that of Comparative Example 18, and Lipidure-BL1003 had an effect of improving the relative ratio equal to or greater than that of Comparative Example 18. Similar results were obtained for the specimen 22. In the specimen 22, both Lipidure-BL1002 and Lipidure-BL1003 exhibited an effect of improving the relative ratio to be equal to or greater than that of Comparative Example 18.

Even SP-D could also inhibit a non-specific reaction caused by the sample in the LTIA reagent to which the compound found in the present application was added, as in Experimental Example 1. That is, the relative ratio of the measured value with respect to the measured value obtained by the CLEIA method, which is a measurement method for performing B/F separation, was improved.

[Experimental Example 5] Use of non-specific reaction inhibitor in pulmonary surfactant protein D (SP-D) measurement reagent - 2

1. Measurement by CLEIA method (Reference Example 4)

1-1. Measurement reagent

Same as in Reference Example 3

1-2. Sample

Human serum specimen 200 specimens (however, the donor of each specimen is different.)

1-3. Measurement procedure

Same as in Reference Example 3

2. Measurement using LTIA method reagent

2-1. Measurement reagents (Comparative Examples 23 to 25 and Example 11)

(1) First reagent

· In Comparative Example 23, the first reagent described in Comparative Example 17 was used.
· In Comparative Example 24, 25 μg/mL of an anti-human IgM antibody was added to the first reagent described in Comparative Example 17.

Regarding the anti-human IgM antibody, the antibody described in Japanese Patent No. 4625879 (microorganism accession number: FERM BP-11134) was used.

· In Comparative Example 25, 100 μg/mL of a commercially available product Heteroblock (OMEGA Biologicals, Inc.) was added to the first reagent described in Comparative Example 17.
· In Example 11, Lipidure-BL1002 was added to the first reagent described in Comparative Example 17 such that the final concentration was 0.1 % (w/v).

(2) Second reagent

The reagent described in Comparative Example 17 was used.

2-2. Calibrator

Same as in Experimental Example 4.

2-3. Sample

Same as 1-2.

3. Measurement method

Same as in Experimental Example 4.

4. Evaluation method

First, the calibrator was measured, and a calibration curve for each test using each reagent was created. Each specimen was measured using the obtained calibration curve, and the concentration was determined.
Next, scatter diagrams (FIG. 2) were created, with the control value according to Reference Example 4 on the axis of abscissa and the measured value of any of Comparative Examples 23 to 25 or Example 11 on the axis of ordinate. The upper part of FIG. 2 is diagrams related to all the measured specimens. The lower part of FIG. 2 is diagrams excerpted for specimens in which the SP-D measured values according to the Reference Examples were 100 ng/mL or less. In addition, the correlation coefficient and the slope of the regression line calculated by the least squares method for each plot are shown in the diagrams.

5. Results

The correlation coefficient of the LTIA measured value with respect to the measured value according to Reference Example 4, which is a measurement method for performing B/F separation, was 0.987 to 0.989 in Comparative Examples 23 to 25, whereas the correlation coefficient was improved to 0.992 in Example 11. In particular, improvement was remarkable in the specimens indicated by the circles with broken lines on the low value side in the figure.

6. Consideration

Even with SP-D, the convergence in a measurement method involving B/F separation improved in the LTIA reagent to which the component of the present invention had been added.
From the above, it was considered that the non-specific reaction inhibitory effect of Lipidure-BL1002, 1003, and 1103 having the property of the present invention is not limited to the measurement items and can be widely applied to the LTIA reagent.

[Experimental Example 6] Use of non-specific reaction inhibitor in soluble interleukin 2 receptor (sIL-2R) measurement reagent - 4

1. Measurement by CLEIA method (Reference Example 5)

1-1. Measurement reagent

Same as in Reference Example 1

1-2. Sample

Human serum specimen Specimen numbers 23, 24, and 25 (however, the donor of each specimen is different.)

1-3. Measurement procedure

Same as in Reference Example 1

2. Measurement by ELISA method (Comparative Examples 26 to 29 and Example 12)

2-1. Method

(1) An anti sIL-2R monoclonal antibody (clone number: 92212) was dissolved in a 20 mM phosphate buffer solution (pH 7.2; hereinafter, referred to as PBS) containing 150 mM sodium chloride to a concentration of 10 μg/mL, 100 μL of the dissolved solution was dispensed into each well of a 96-well microplate, and the plate was left to stand at 4°C overnight.
(2) Each above-described well was washed three times with 400 μL of PBS containing 0.05% (w/v) Tween (registered trademark) 20 (hereinafter, referred to as PBST), subsequently 200 μL of PBST containing 1% (w/v) bovine serum albumin (hereinafter, referred to as BSA-PBST) was added thereto, and blocking was carried out at room temperature for 1 hour. This was used as a plate for ELISA.
(3) Each well of the above-described plate for ELISA was washed three times with 400 μL of PBST, subsequently 100 μL of a sample diluted 40-fold with a specimen diluent obtained by adding the components described in Table 7 to BSA-PBST was added to each above-described well, and the plate was left to stand at room temperature for 1 hour.
(4) Each above-described well was washed three times with 400 μL of PBST, subsequently 100 μL of a biotin-labeled anti sIL-2R monoclonal antibody (clone number: 92204R) diluted to 0.50 μg/mL with the specimen diluent described in Table 7 was dispensed into each above-described well, and the plate was left to stand at room temperature for 1 hour.
(5) Each above-described well was washed three times with 400 μL of PBST, subsequently 100 μL of HRP-labeled streptavidin (Thermo Fisher Scientific, Inc.) diluted to 0.20 μg/mL with BSA-PBST was dispensed into each above-described well, and the plate was left to stand at room temperature for 30 minutes.
(6) After washing each above-described well three times with 400 μL of PBST, 50 μL of a citrate buffer solution (pH 5.0) containing 0.2% (w/v) orthophenylenediamine and 0.02% (w/v) hydrogen peroxide was added thereto, the plate was left to stand at room temperature for 10 minutes, subsequently 50 μL of 4.5 N sulfuric acid was added thereto to stop

the enzymatic reaction, and the absorbance at a wavelength of 492 nm was measured.
(7) A standard substance having a known concentration was used as a calibrator, and the sIL-2R value in the test sample was calculated.

[Table 7]

| | Additive | Concentration (wt%) |
|---|---|---|
| Comparative Example 26 | Not added | - |
| Comparative Example 27 | Lipidure-BL206 | 0.10% |
| Comparative Example 28 | Lipidure-BL802 | 0.10% |
| Comparative Example 29 | Lipidure-BL1301 | 0.10% |
| Example 12 | Lipidure-BL1002 | 0.10% |

2-2. Sample

Same as 1-2.

3. Evaluation method

A calibration curve for each test using each reagent was created by using the measurement results of the calibrator. The measured value of each specimen was determined using the obtained calibration curve. When the measurement sensitivity of Comparative Example 26 was set to 100%, the measurement sensitivities of Comparative Examples 27 and 28 and Example 12 were 106% to 108%, which were roughly equivalent to that of Comparative Example 26. The sensitivity of Comparative Example 29 was 117%.

For each specimen, a value obtained by dividing the measured value in each of Comparative Examples 26 to 29 and Example 12 by the measured value of Reference Example 5 was calculated and evaluated as a relative ratio (%). In a case where the relative ratio was 90% or more and 110% or less, it was determined that the measured value was not deviated from the measured value of Reference Example 5. The obtained results are shown in Table 8.

4. Results

(1) Control specimen

Specimens 23 and 24, in which the proportion (relative ratio) of the measured value of the ELISA method in which no additive was added (Comparative Example 26), with respect to the measured value of the CLEIA method (Reference Example 5) was 108% to 109%, were evaluated as "control specimens" in which a non-specific reaction did not occur in the ELISA method. In the control specimens, the relative ratio with respect to Reference Example 5 was 106% even in Example 12, and a non-specific reaction did not occur.

(2) Deviation specimen

Specimen 25, in which the proportion (relative ratio) of the measured value of the ELISA method in which no additive was added (Comparative Example 26), with respect to the measured value of the CLEIA method (Reference Example 5) was 114%, was evaluated as a "deviation specimen" in which a non-specific reaction had occurred in the ELISA method.

In a case where 0.1 % (w/v) of Lipidure that does not have the property of the present invention was added (Comparative Examples 27 to 29), the relative ratio was 114%, and the deviation was not improved. On the other hand, in the ELISA method in which 0.1% (w/v) of Lipidure-BL1002 having the property of the present invention was added (Example 12), the relative ratio was 101%, and the relative ratio with respect to Reference Example 5 was improved.

[Table 8]

| Specimen No. | IL-2R measurement Relative ratio (%) | | |
|---|---|---|---|
| | Control specimen | | Deviation specimen |
| | 23 | 24 | 25 |
| Comparative Example 26 | 109% | 108% | 114% |
| Comparative Example 27 | 105% | 105% | 114% |
| Comparative Example 28 | 109% | 100% | 114% |
| Comparative Example 29 | 107% | 112% | 114% |
| Example 12 | 106% | 106% | 101% |

5. Consideration

Even in the ELISA reagent, which is a measurement method involving B/F separation, when the component of the present invention was added to the specimen diluent, the consistency with the measured value of the CLEIA method, which is a measurement method involving B/F separation, was improved.

From the above, it was considered that the non-specific reaction inhibitory effect provided by Lipidure-BL1002, 1003, and 1103 that have the property of the present invention is not limited to measurement methods without B/F separation and can be widely applied to biochemical measurement methods.

## Claims

**1.** A non-specific reaction inhibitor, which is a copolymer comprising a constitutional unit A derived from 2-methacryloyloxyethyl phosphorylcholine and exhibiting the following physical property X,

wherein the non-specific reaction inhibitor is used in a use application of inhibiting a non-specific reaction when measuring a measurement target substance contained in a biological sample by mixing the copolymer with the biological sample,
<physical property X>
in a spectrum of Fourier transform infrared spectroscopy according to an attenuated total reflection method (ATR method), there is a peak having a maximum absorption at 2870 to 2833 $cm^{-1}$.

**2.** A method for using a non-specific reaction inhibitor, the method comprising: using a copolymer comprising a constitutional unit A derived from 2-methacryloyloxyethyl phosphorylcholine and exhibiting the following physical property X, as a non-specific reaction inhibitor,

wherein a non-specific reaction is inhibited when measuring a measurement target substance contained in a biological sample by mixing the copolymer with the biological sample,
<physical property X>
in a spectrum of Fourier transform infrared spectroscopy according to an attenuated total reflection method (ATR method), there is a peak having a maximum absorption at 2870 to 2833 $cm^{-1}$.

**3.** A method for inhibiting a non-specific reaction, the method comprising:

inhibiting a non-specific reaction when measuring a measurement target substance contained in a biological sample by mixing a copolymer comprising a constitutional unit A derived from 2-methacryloyloxyethyl phosphorylcholine and exhibiting the following physical property X, with the biological sample,
<physical property X>
in a spectrum of Fourier transform infrared spectroscopy according to an attenuated total reflection method (ATR method), there is a peak having a maximum absorption at 2870 to 2833 $cm^{-1}$.

**4.** A biochemical measurement reagent, which is a reagent used for biochemical measurement,

wherein the reagent contains a copolymer comprising a constitutional unit A derived from 2-methacryloyloxyethyl

phosphorylcholine and exhibiting the following physical property X,
<physical property X>
in a spectrum of Fourier transform infrared spectroscopy according to an attenuated total reflection method (ATR method), there is a peak having a maximum absorption at 2870 to 2833 $cm^{-1}$.

**5.** A biochemical measurement reagent, which is a reagent used for biochemical measurement,

wherein the reagent contains a copolymer comprising a constitutional unit A derived from 2-methacryloyloxyethyl phosphorylcholine and exhibiting the following physical property X, and
the copolymer is contained as a non-specific reaction inhibitor that inhibits a non-specific reaction in a biological sample,
<physical property X>
in a spectrum of Fourier transform infrared spectroscopy according to an attenuated total reflection method (ATR method), there is a peak having a maximum absorption at 2870 to 2833 $cm^{-1}$.

**6.** The biochemical measurement reagent according to Claim 4 or 5, wherein the biochemical measurement is a homogeneous method.

**7.** The biochemical measurement reagent according to Claim 5, wherein the biochemical measurement is a measurement based on an antigen-antibody reaction.

**8.** The biochemical measurement reagent according to Claim 4 or 5, wherein the biochemical measurement is a latex turbidimetric immunoassay.

**9.** The biochemical measurement reagent according to Claim 4 or 5, wherein a biological sample as a measurement target of the biochemical measurement is a blood sample.

**10.** The biochemical measurement reagent according to Claim 4 or 5, wherein the biochemical measurement is detection of an antigen-antibody reaction between any one or more selected from a soluble interleukin 2 receptor and a pulmonary surfactant protein D, which are contained in a biological sample as a measurement target, and an antibody added to the biological sample.

**11.** A specimen pretreatment solution, which is a reagent used for biochemical measurement,

wherein the reagent contains a copolymer comprising a constitutional unit A derived from 2-methacryloyloxyethyl phosphorylcholine and exhibiting the following physical property X, and a liquid medium in which the copolymer is dissolved or dispersed,
<physical property X>
in a spectrum of Fourier transform infrared spectroscopy according to an attenuated total reflection method (ATR method), there is a peak having a maximum absorption at 2870 to 2833 $cm^{-1}$.

**12.** A biochemical measurement reagent kit, comprising:
the biochemical measurement reagent according to Claim 4 or 5.

FIG. 1A

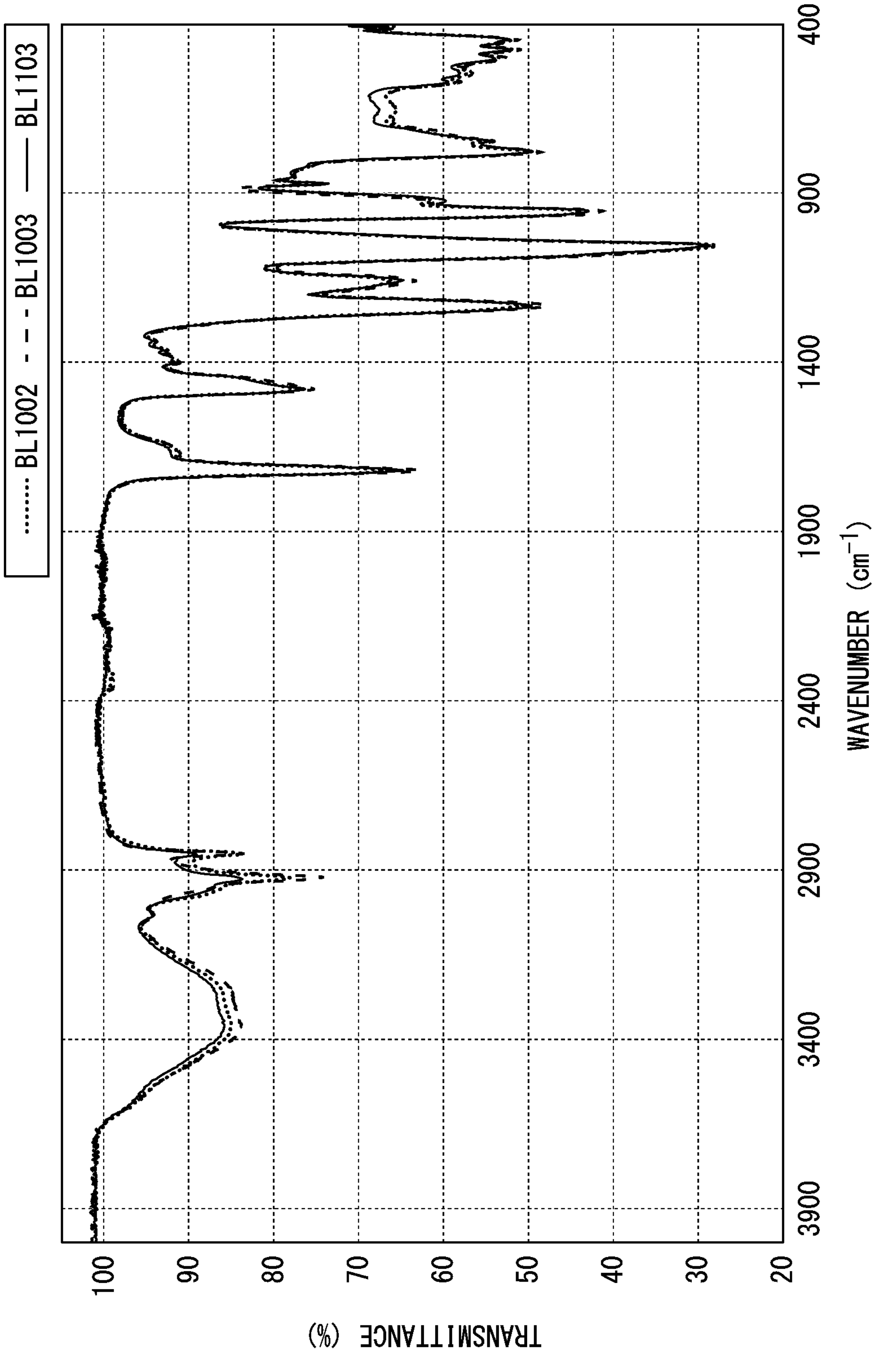
BL1002
BL1003
BL1103
TRANSMITTANCE (%)
WAVENUMBER (cm-1)
100
90
80
70
60
50
40
30
20
3900
3400
2900
2400
1900
1400
900
400

FIG. 1B

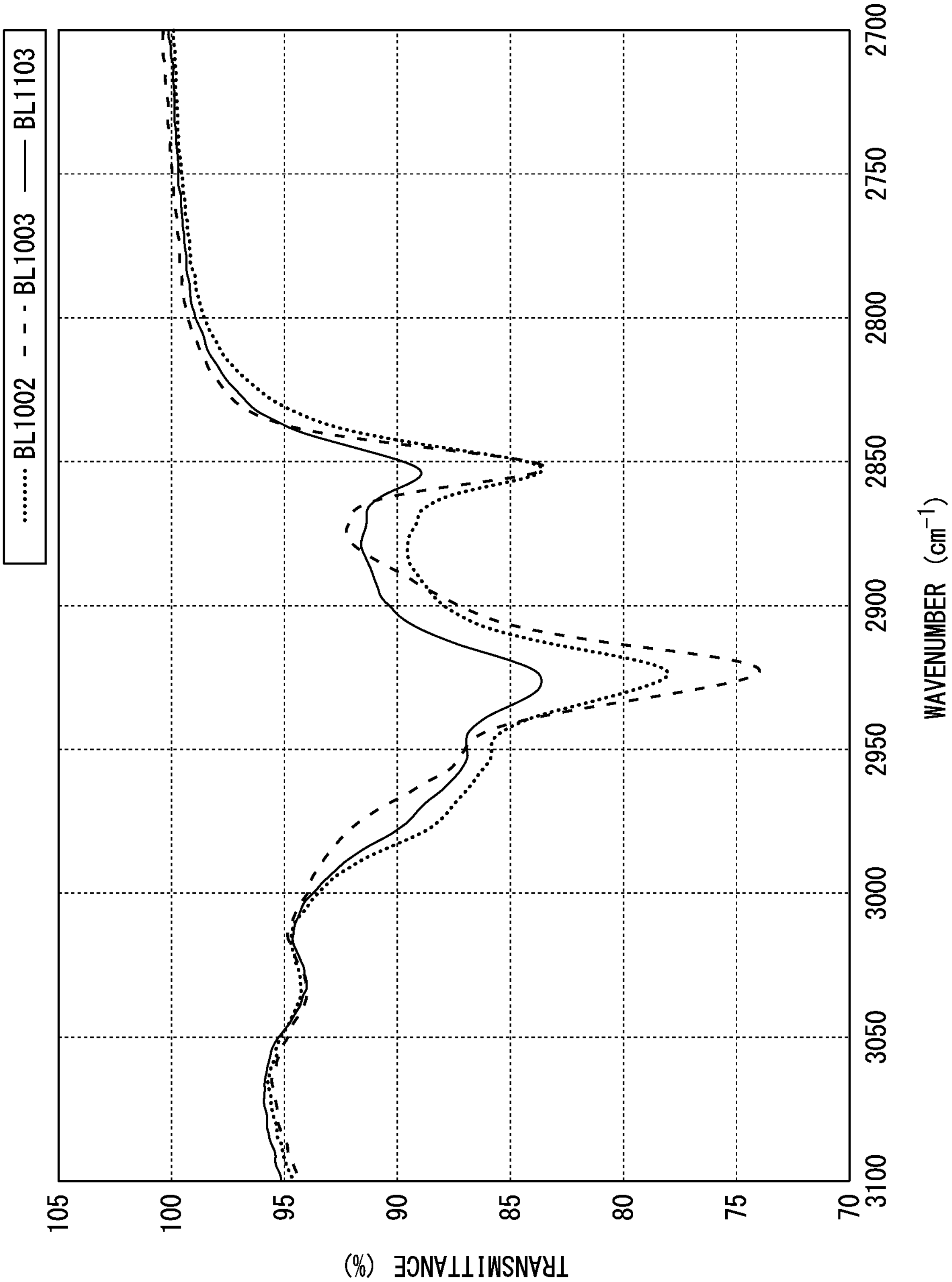

BL1002
BL1003
BL1103
TRANSMITTANCE (%)
105
100
95
90
85
80
75
70
3100
3050
3000
2950
2900
2850
2800
2750
2700
WAVENUMBER (cm-1)

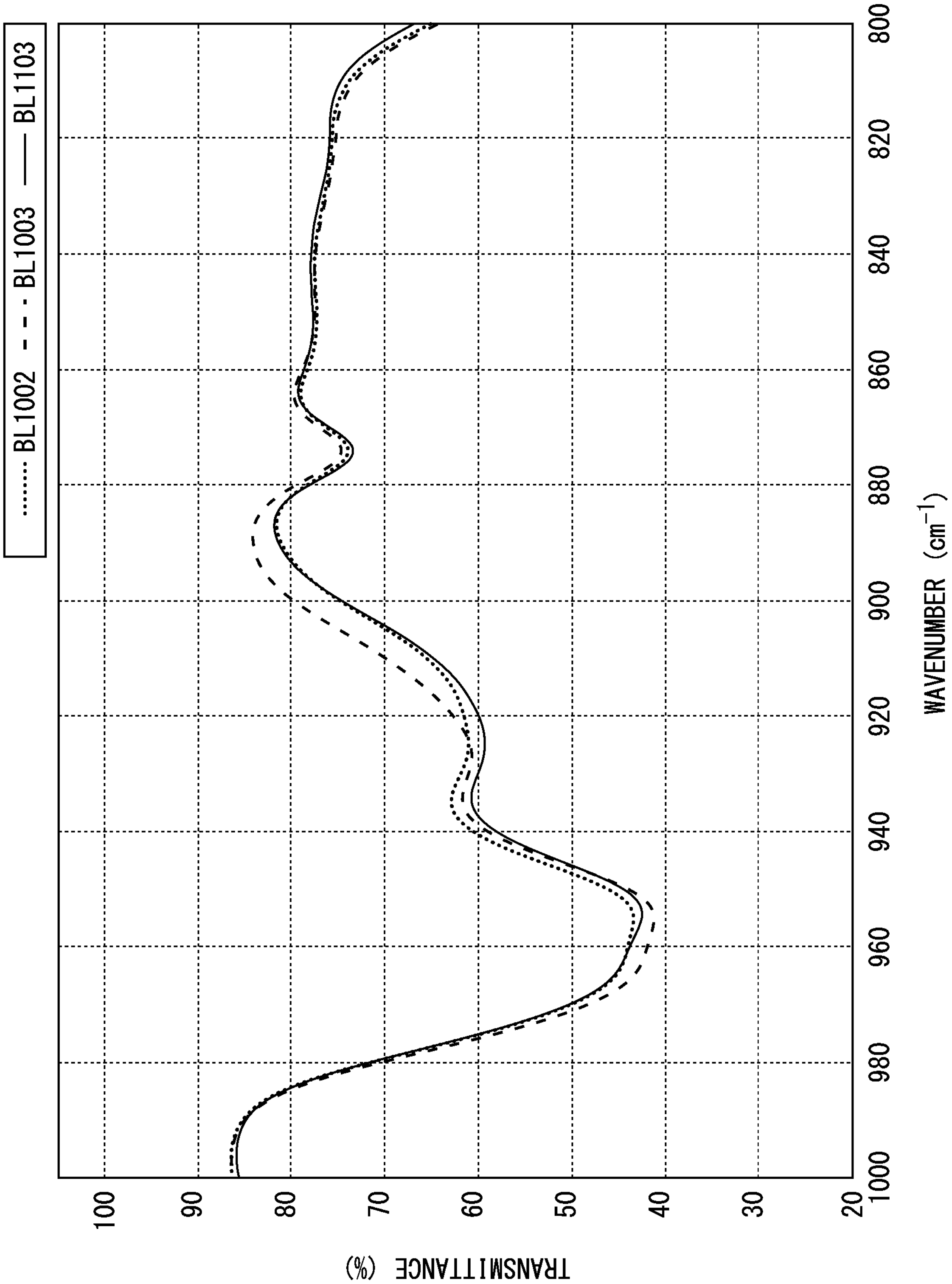
BL1002
BL1003
BL1103
TRANSMITTANCE (%)
100
90
80
70
60
50
40
30
20
1000
980
960
940
920
900
880
860
840
820
800
WAVENUMBER (cm$^{-1}$)

FIG. 1C

FIG. 1D

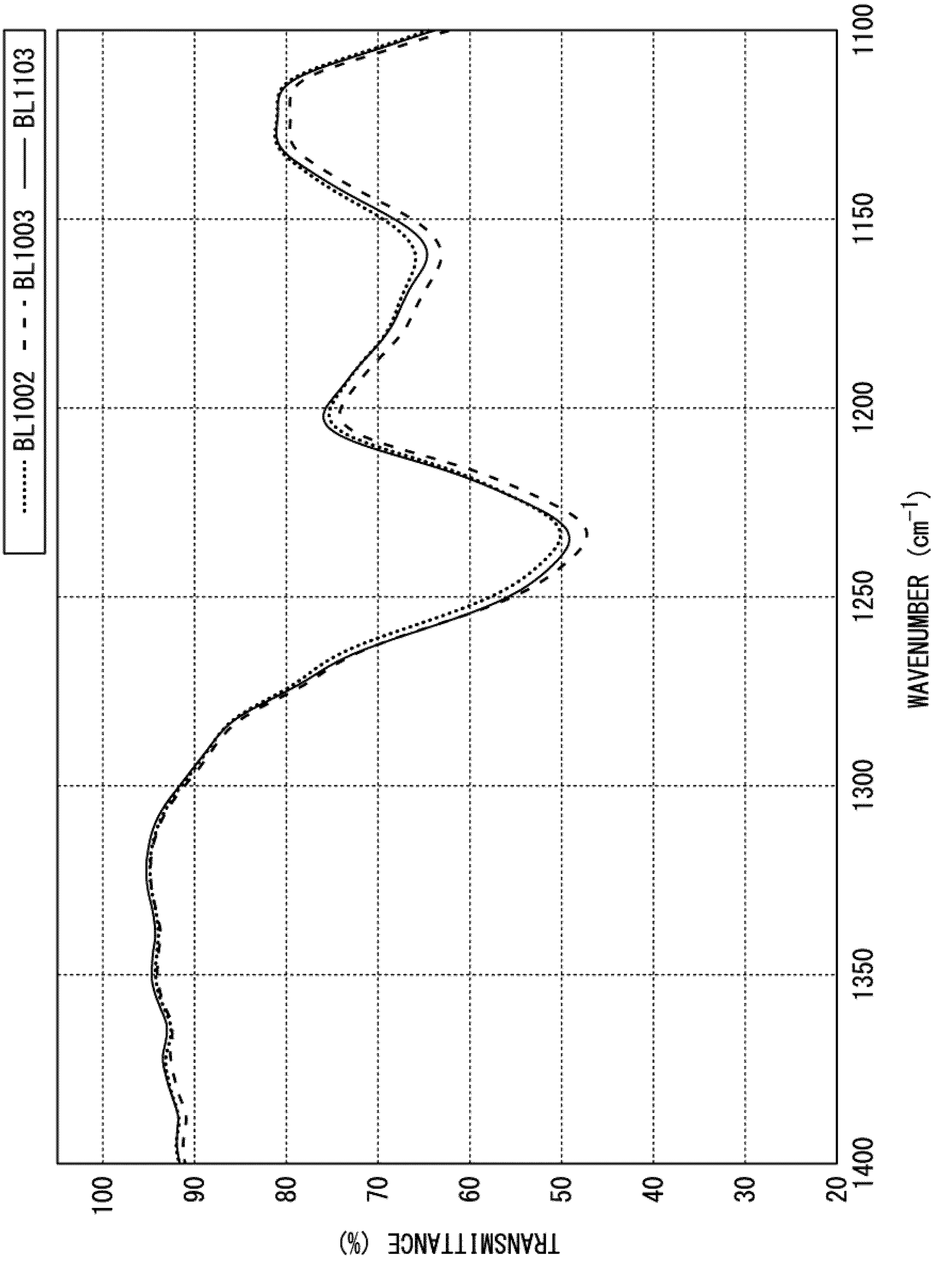
BL1002
BL1003
BL1103
TRANSMITTANCE (%)
WAVENUMBER (cm$^{-1}$)
100
90
80
70
60
50
40
30
20
1400
1350
1300
1250
1200
1150
1100

FIG. 1E

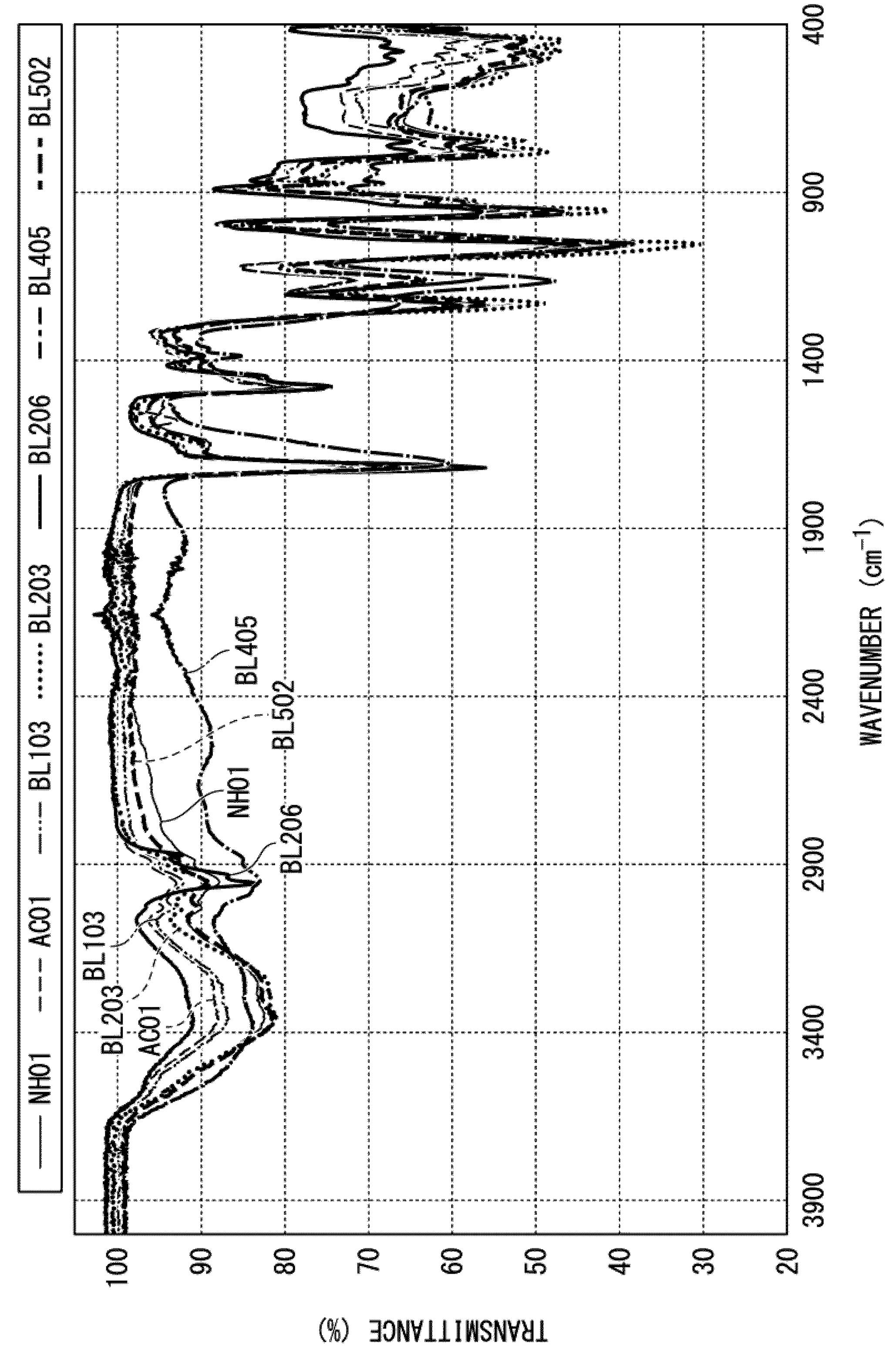

NH01
AC01
BL103
BL203
BL206
BL405
BL502
TRANSMITTANCE (%)
WAVENUMBER (cm$^{-1}$)
100
90
80
70
60
50
40
30
20
3900
3400
2900
2400
1900
1400
900
400

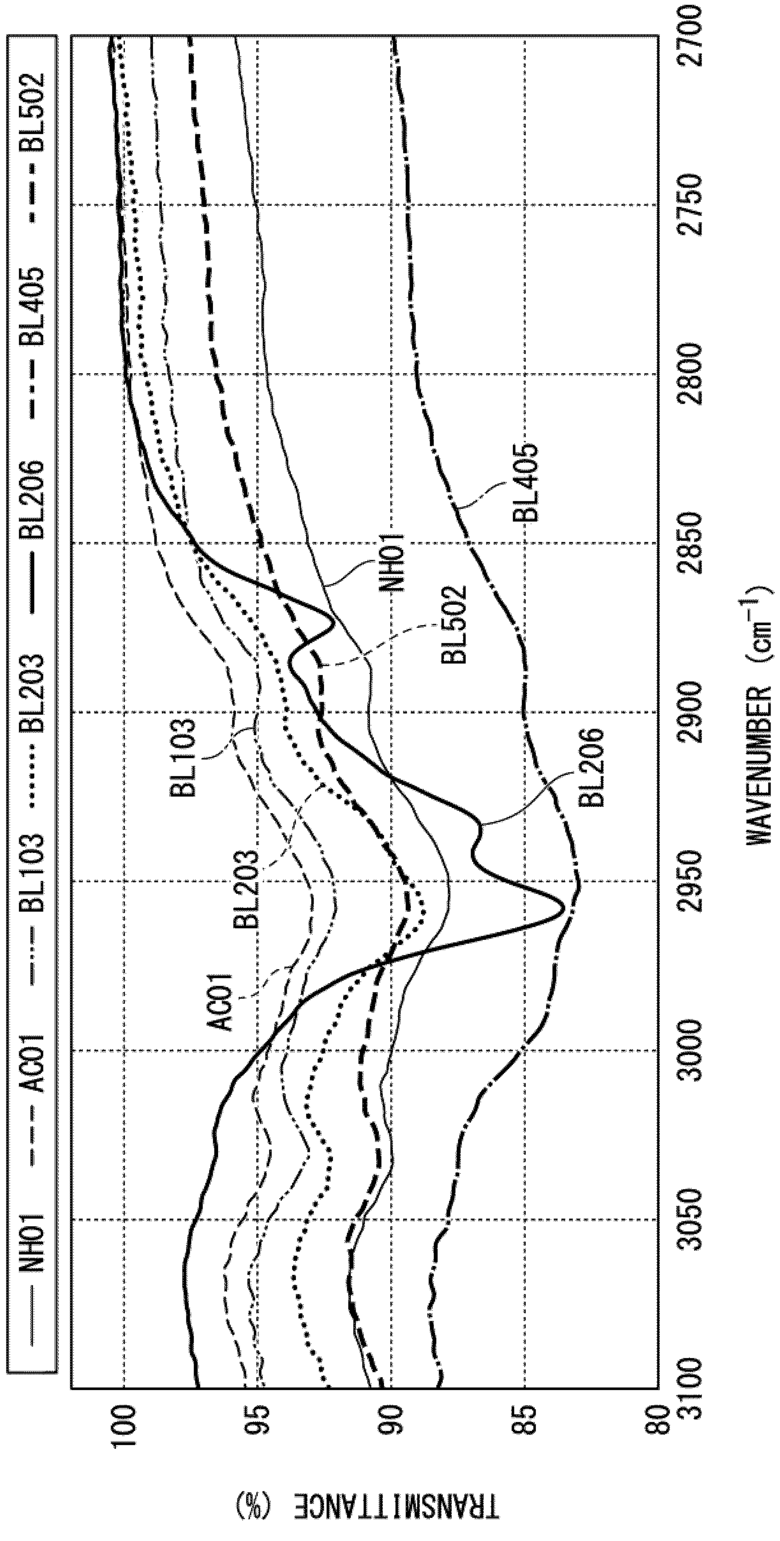
NH01
AC01
BL103
BL203
BL206
BL405
BL502
TRANSMITTANCE (%)
100
95
90
85
80
3100
3050
3000
2950
2900
2850
2800
2750
2700
WAVENUMBER (cm$^{-1}$)
AC01
BL203
BL103
BL206
BL502
NH01
BL405

FIG. 1F

FIG. 1G

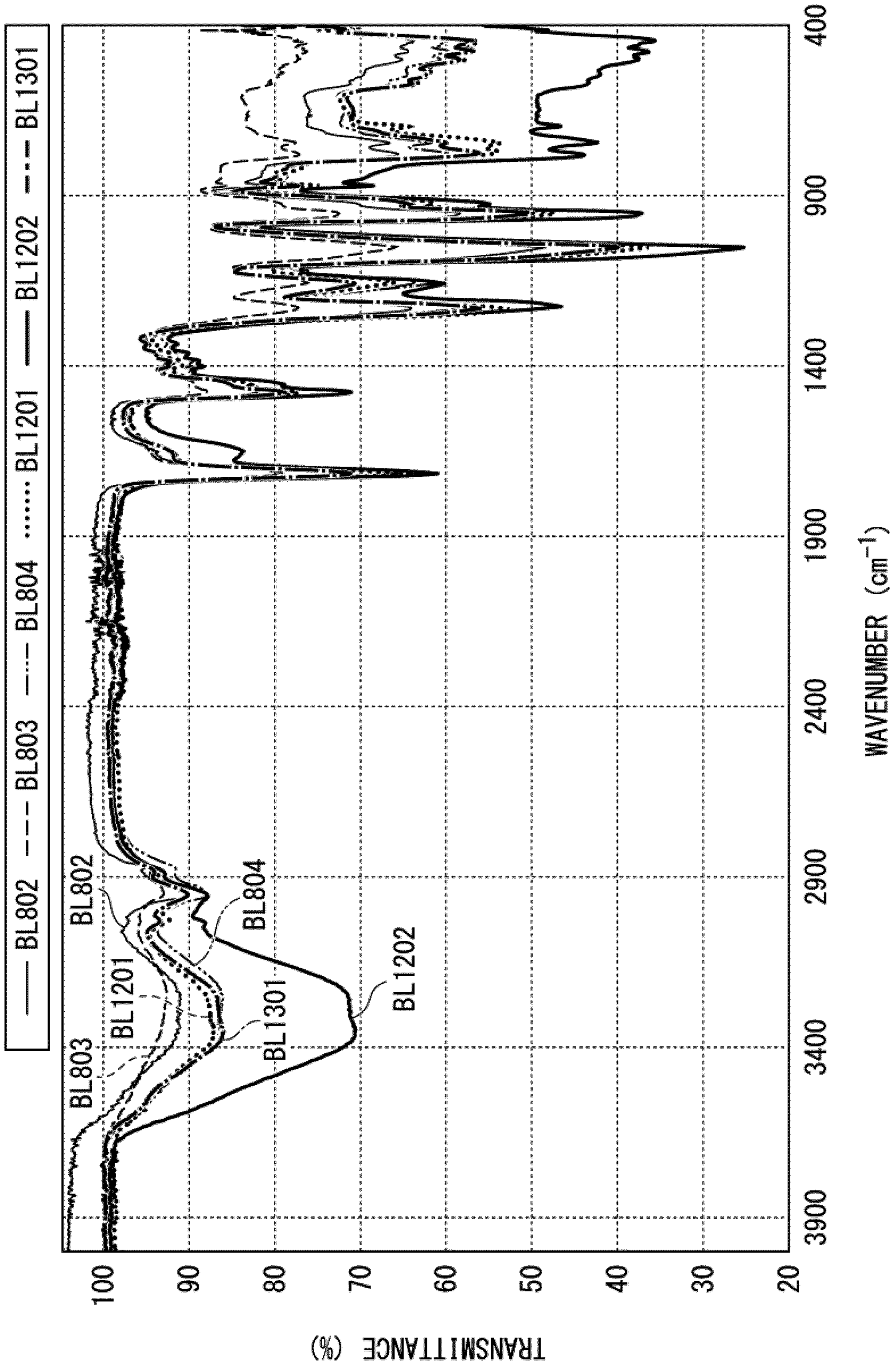
BL802
BL803
BL804
BL1201
BL1202
BL1301
TRANSMITTANCE (%)
100
90
80
70
60
50
40
30
20
3900
3400
2900
2400
1900
1400
900
400
WAVENUMBER (cm$^{-1}$)

FIG. 1H

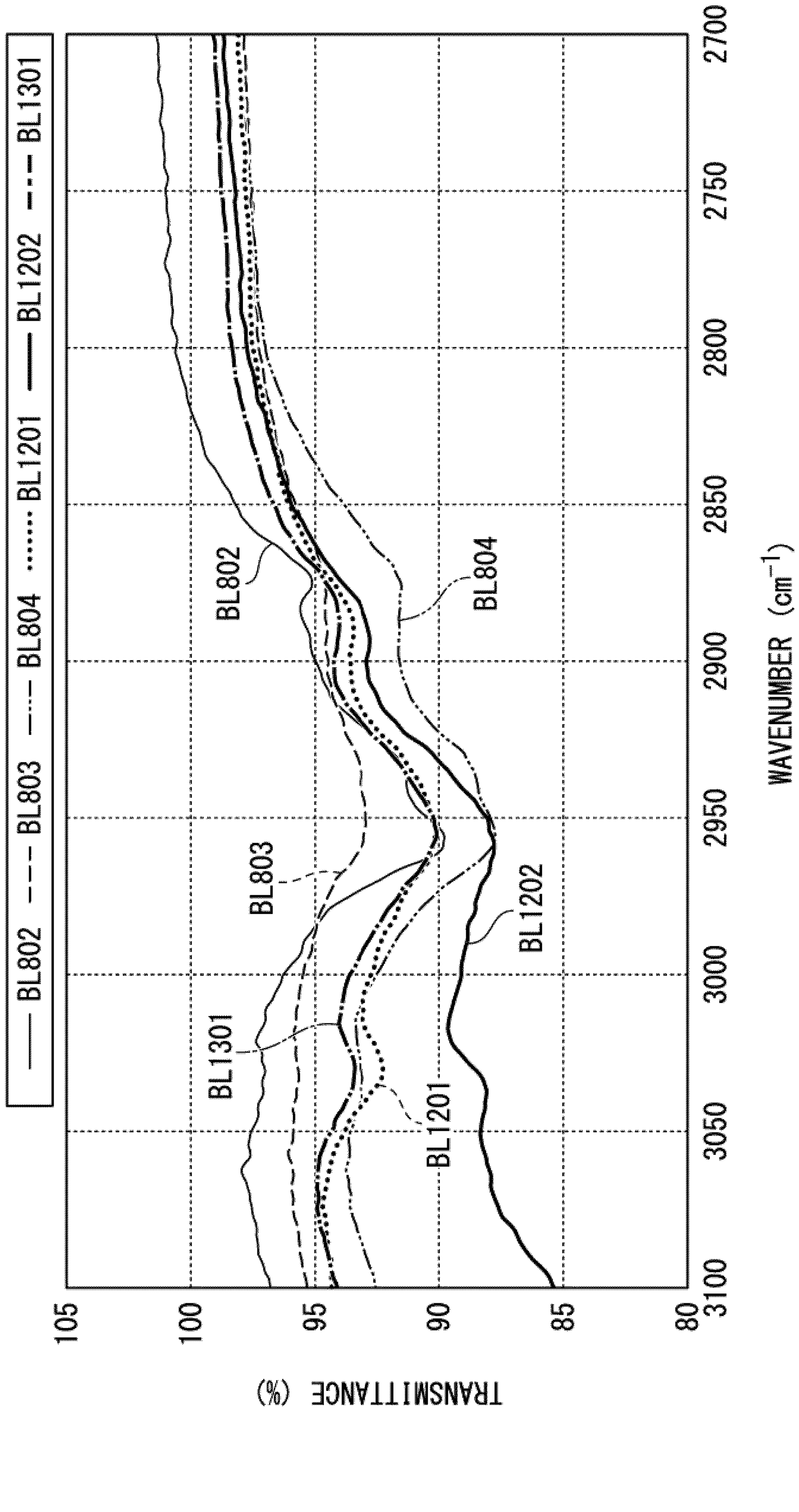
BL802
BL803
BL804
BL1201
BL1202
BL1301
TRANSMITTANCE (%)
105
100
95
90
85
80
3100
3050
3000
2950
2900
2850
2800
2750
2700
WAVENUMBER (cm$^{-1}$)

FIG. 2

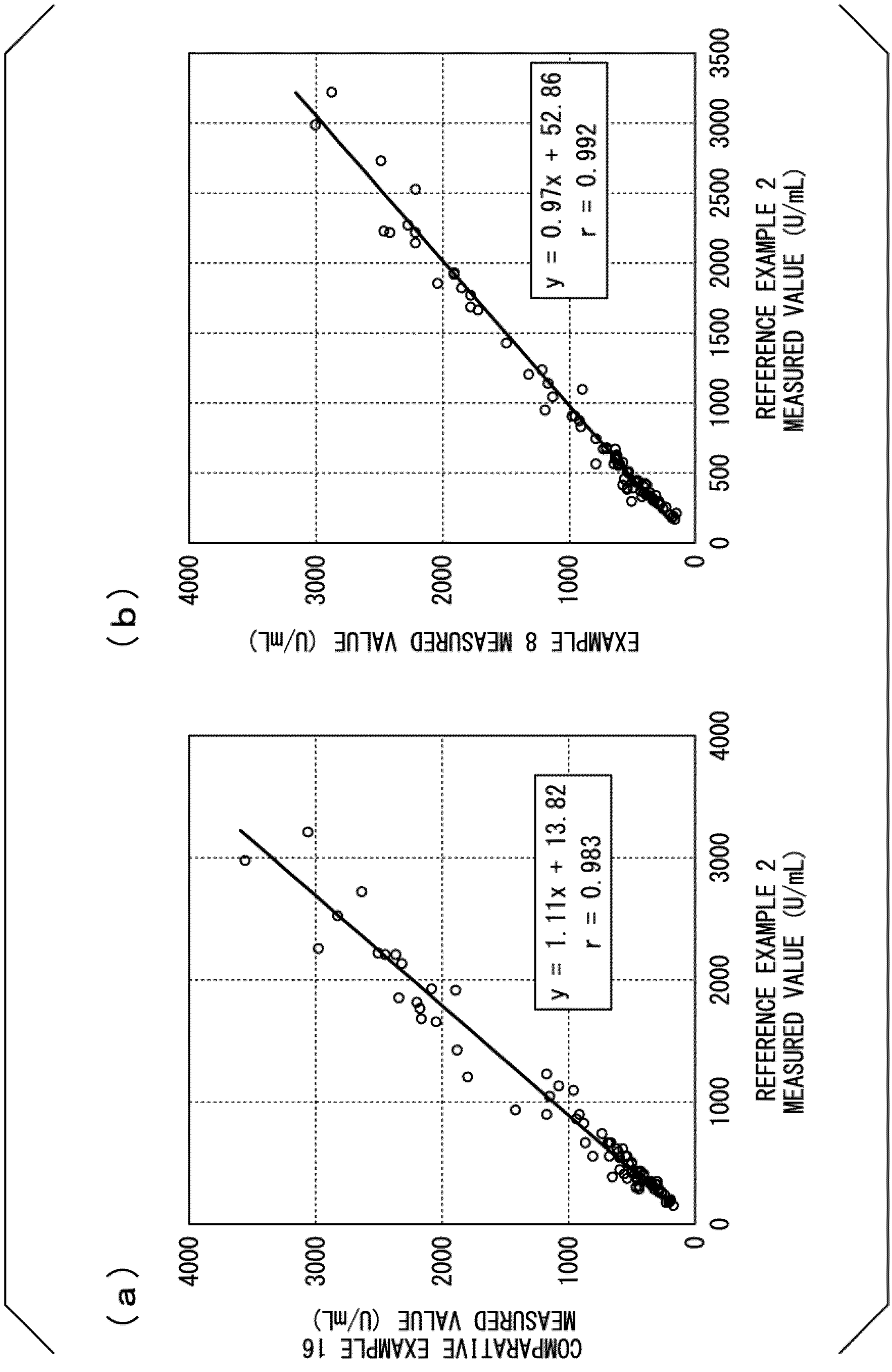
(a)
y = 1.11x + 13.82
r = 0.983
COMPARATIVE EXAMPLE 16 MEASURED VALUE (U/mL)
REFERENCE EXAMPLE 2 MEASURED VALUE (U/mL)
0
1000
2000
3000
4000
(b)
y = 0.97x + 52.86
r = 0.992
EXAMPLE 8 MEASURED VALUE (U/mL)
REFERENCE EXAMPLE 2 MEASURED VALUE (U/mL)
0
500
1000
1500
2000
2500
3000
3500
4000

FIG. 3

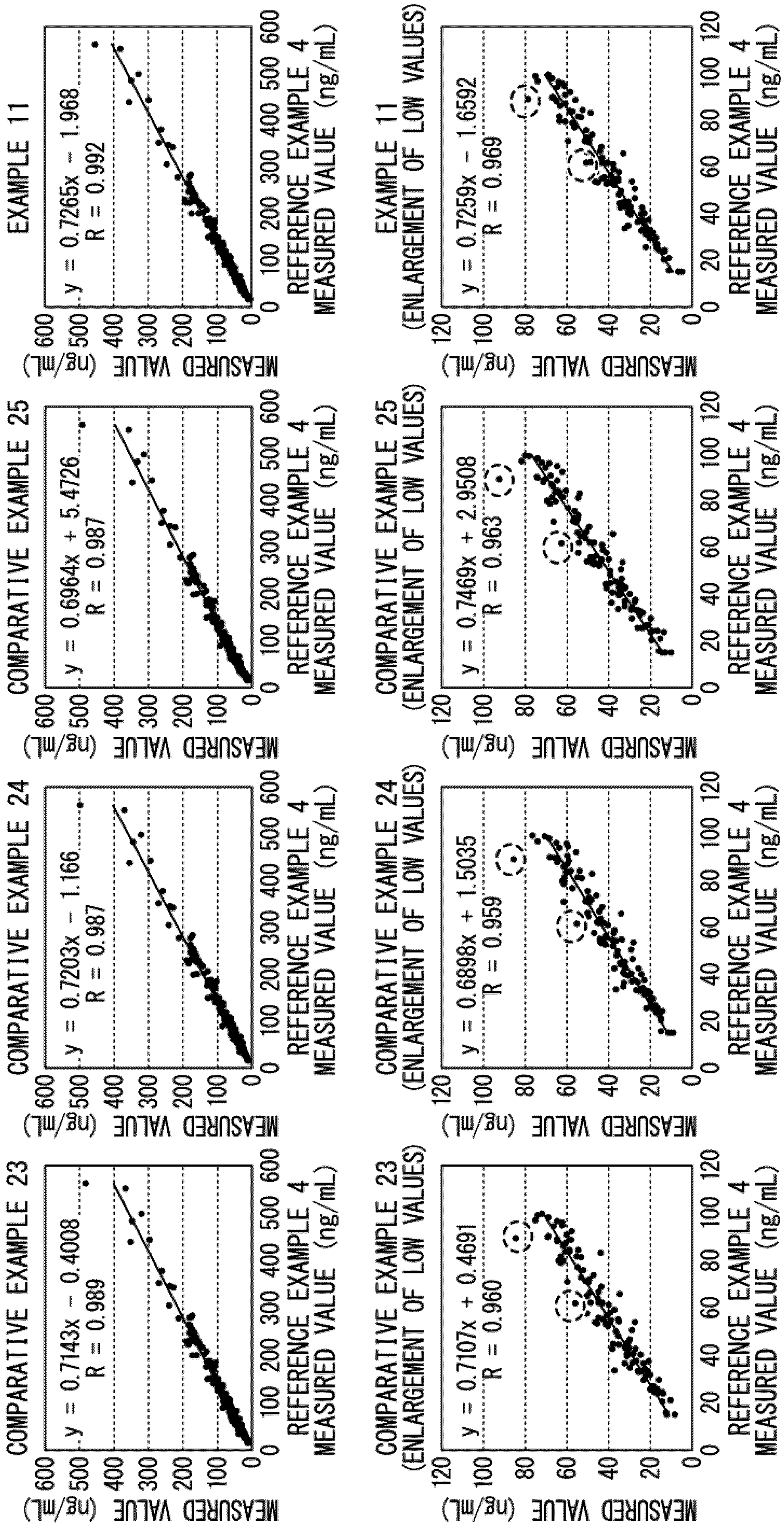
COMPARATIVE EXAMPLE 23
y = 0.7143x − 0.4008
R = 0.989
COMPARATIVE EXAMPLE 24
y = 0.7203x − 1.166
R = 0.987
COMPARATIVE EXAMPLE 25
y = 0.6964x + 5.4726
R = 0.987
EXAMPLE 11
y = 0.7265x − 1.968
R = 0.992
COMPARATIVE EXAMPLE 23 (ENLARGEMENT OF LOW VALUES)
y = 0.7107x + 0.4691
R = 0.960
COMPARATIVE EXAMPLE 24 (ENLARGEMENT OF LOW VALUES)
y = 0.6898x + 1.5035
R = 0.959
COMPARATIVE EXAMPLE 25 (ENLARGEMENT OF LOW VALUES)
y = 0.7469x + 2.9508
R = 0.963
EXAMPLE 11 (ENLARGEMENT OF LOW VALUES)
y = 0.7259x − 1.6592
R = 0.969
MEASURED VALUE (ng/mL)
REFERENCE EXAMPLE 4 MEASURED VALUE (ng/mL)
0
100
200
300
400
500
600
20
40
60
80
100
120

## INTERNATIONAL SEARCH REPORT

International application No.
**PCT/JP2023/036644**

**A. CLASSIFICATION OF SUBJECT MATTER**

***G01N 33/531***(2006.01)i; ***C08F 220/10***(2006.01)i; ***C08F 230/02***(2006.01)i; ***G01N 33/53***(2006.01)i; ***G01N 33/542***(2006.01)i; ***G01N 33/543***(2006.01)i; ***G01N 33/545***(2006.01)i
FI: G01N33/531 B; G01N33/542; G01N33/543 581J; G01N33/543 583; G01N33/545 B; G01N33/53 D; C08F230/02; C08F220/10

According to International Patent Classification (IPC) or to both national classification and IPC

**B. FIELDS SEARCHED**

Minimum documentation searched (classification system followed by classification symbols)

G01N33/531; C08F220/10; C08F230/02; G01N33/53; G01N33/542; G01N33/543; G01N33/545

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | 診断薬分野製品｜ライフサイエンス事業. 日油株式会社. [online], 05 March 2016 [retrieved on 01 December 2023], Internet: <URL: https://www.nof.co.jp/business/life/mpc-product04>, (Life Science. NOF CORPORATION), non-official translation (Diagnostic Agent Products.) Diagnostic Agent Additives (LIPIDURE-BL Usage Cases: BL1000 Series), Blocking Effect (Ex. 3: Magnetic Fine Particle Blocking (Antibody Diluent)), Sensitizing Effect (Ex. 1: Sensitizing Effect in Latex Agglutination) | 1-9, 11-12 |
| Y | | 1-12 |
| X | Additives for Diagnostics. NOF CORPORATION. [online], 02 December 2020 [retrieved on 01 December 2023], Internet: <URL: https://www.nofamerica.com/store/images/companies/1/images/Biolipidure.pdf?1606998287683> Product Line of Biolipidure, pp. 5, 8 | 1-9, 11-12 |
| Y | | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 December 2023** | **19 December 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/036644**

**C. DOCUMENTS CONSIDERED TO BE RELEVANT**

| Category* | Citation of document, with indication, where appropriate, of the relevant passages | Relevant to claim No. |
|---|---|---|
| Y | JP 2021-96268 A (HITACHI CHEMICAL DIAGNOSTICS SYSTEMS CO., LTD.) 24 June 2021 (2021-06-24) abstract, claims, paragraphs [0013], [0025] | 10 |
| Y | JP 2020-186912 A (JSR CORP.) 19 November 2020 (2020-11-19) abstract, claims, paragraph [0089] | 1-12 |
| A | 小林吉倫. Lipidure-BL添加によるブタ精子のスライドガラスへの付着低減. 第106回日本繁殖生物学会大会要旨集. 10 September 2013, p. P-70, non-official translation (KOBAYASHI, Yoshitomo. Using Libidure-BL Additive to Reduce Adhesion of Boar Sperm to Slide Glass. Proceedings of the 106th Meeting of the Society for Reproduction and Development.) entire text | 1-12 |
| A | JP 2019-27957 A (SEKISUI MEDICAL CO., LTD.) 21 February 2019 (2019-02-21) examples | 1-12 |
| A | JP 2020-509349 A (ABAXIS, INC.) 26 March 2020 (2020-03-26) claims 1, 17, 18, 66, paragraph [0015] | 1-12 |
| A | WO 2016/017037 A1 (BL KK) 04 February 2016 (2016-02-04) claims 9-12, paragraph [0072] | 1-12 |
| A | JP 2018-124277 A (SANYO CHEMICAL INDUSTRIES, LTD.) 09 August 2018 (2018-08-09) paragraphs [0034], [0038]-[0040] | 1-12 |
| A | WO 2016/136918 A1 (SEKISUI MEDICAL CO., LTD.) 01 September 2016 (2016-09-01) examples | 1-12 |
| A | JP 4544437 B2 (SEKISUI MEDICAL CO., LTD.) 15 September 2010 (2010-09-15) claims | 1-12 |
| A | CN 109765382 A (BEIJING STRONG BIOTECHNOLOGIES, INC.) 17 May 2019 (2019-05-17) abstract, claims | 1-12 |
| A | JP 2001-228149 A (NOF CORP.) 24 August 2001 (2001-08-24) claims | 1-12 |
| A | JP 2001-318099 A (KYOWA MEDEX CO., LTD.) 16 November 2001 (2001-11-16) abstract | 1-12 |
| A | WO 2002/018953 A1 (KYOWA MEDEX CO., LTD.) 07 March 2002 (2002-03-07) abstract, claims | 1-12 |
| A | JP 2015-148558 A (KYOTO INSTITUTE OF TECHNOLOGY) 20 August 2015 (2015-08-20) abstract, claims | 1-12 |
| A | JP 4629564 B2 (SEKISUI MEDICAL CO., LTD.) 09 February 2011 (2011-02-09) paragraphs [0008], [0009] | 1-12 |
| P, X | JP 2022-152733 A (SEKISUI MEDICAL CO., LTD.) 12 October 2022 (2022-10-12) claims, paragraph [0018] | 1-12 |
| P, X | JP 2023-133205 A (SANYO CHEMICAL INDUSTRIES, LTD.) 22 September 2023 (2023-09-22) paragraphs [0051]-[0053] | 1-12 |

Form PCT/ISA/210 (second sheet) (January 2015)

**INTERNATIONAL SEARCH REPORT**
**Information on patent family members**

International application No.
**PCT/JP2023/036644**

| Patent document cited in search report | Publication date (day/month/year) | Patent family member(s) | Publication date (day/month/year) |
|---|---|---|---|
| JP 2021-96268 A | 24 June 2021 | JP 2017-134067 A | |
| JP 2020-186912 A | 19 November 2020 | WO 2019/031581 A1 | |
| JP 2019-27957 A | 21 February 2019 | (Family: none) | |
| JP 2020-509349 A | 26 March 2020 | WO 2018/140953 A1<br>paragraph [0014], claims 1, 17, 18, 66<br>JP 2023-99008 A<br>US 2022/0091113 A1<br>EP 3574308 A1<br>KR 10-2019-0112783 A<br>CN 110892250 A<br>KR 10-2023-0011477 A | |
| WO 2016/017037 A1 | 04 February 2016 | US 2016/0202251 A1<br>paragraph [0084], claims<br>CN 105492605 A | |
| JP 2018-124277 A | 09 August 2018 | (Family: none) | |
| WO 2016/136918 A1 | 01 September 2016 | EP 3264085 A1<br>example<br>CN 107533055 A<br>US 2018/0113127 A1 | |
| JP 4544437 B2 | 15 September 2010 | EP 2261661 A1<br>claims<br>US 2011/0027912 A1<br>WO 2009/123060 A1<br>CN 101978268 A | |
| CN 109765382 A | 17 May 2019 | (Family: none) | |
| JP 2001-228149 A | 24 August 2001 | (Family: none) | |
| JP 2001-318099 A | 16 November 2001 | US 2001/0026927 A1<br>abstract<br>EP 1130396 A1<br>KR 10-2001-0085485 A<br>CN 1311438 A | |
| WO 2002/018953 A1 | 07 March 2002 | EP 1314982 A1<br>abstract, claims<br>JP 4733335 B2<br>US 2003/0166302 A1<br>KR 10-0778102 B1<br>CN 1449495 A | |
| JP 2015-148558 A | 20 August 2015 | (Family: none) | |
| JP 4629564 B2 | 09 February 2011 | EP 1956373 A1<br>paragraphs [0011], [0012]<br>US 2009/0246884 A1<br>WO 2007/066731 A1<br>KR 10-2008-0077357 A<br>CN 101341408 A<br>CN 102565397 A | |
| JP 2022-152733 A | 12 October 2022 | (Family: none) | |
| JP 2023-133205 A | 22 September 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022161609 A
- JP H07012818 A
- JP 2001228149 A
- JP 2001318099 A
- JP 2002022740 A
- WO 2002018953 A
- JP 2015148558 A
- JP 4629564 B
- JP 2002143294 A
- JP H339309 A
- JP H10324634 A
- WO 2022124288 A
- JP 2017181377 A
- JP 2018173429 A
- JP 4625879 B

### Non-patent literature cited in the description

- *Biophysical Chemistry*, 2007, vol. 51, 223-226